(19) **Europäisches Patentamt / European Patent Office / Office européen des brevets**

(11) **EP 4 671 319 A2**

(12) **EUROPEAN PATENT APPLICATION**

(43) Date of publication:
**31.12.2025 Bulletin 2026/01**

(21) Application number: **25216421.5**

(22) Date of filing: **18.04.2024**

(51) International Patent Classification (IPC):
*C08K 5/053* (2006.01)

(52) Cooperative Patent Classification (CPC):
(C-Sets available)
**A61K 9/0051; A61K 9/7007; A61K 31/4178; A61K 47/32; C08L 29/04;** C08K 5/0016; C08L 2203/02; C08L 2205/025; C08L 2207/32; C08L 2207/324 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.04.2023 US 202363462269 P**
**30.04.2023 US 202363463034 P**

(62) Document number(s) of the earlier application(s) in accordance with Art. 76 EPC:
**24725964.1 / 4 547 753**

(71) Applicant: **ABLE Tx Ltd.**
**6971017 Tel-Aviv (IL)**

(72) Inventors:
• **BETSER, Nir**
  **6972201 Tel-Aviv (IL)**
• **ADIN, Itai**
  **8468908 Beer-Sheva (IL)**
• **HADAR, Keren**
  **5848233 Holon (IL)**

(74) Representative: **Mathys & Squire**
**The Shard**
**32 London Bridge Street**
**London SE1 9SG (GB)**

Remarks:
This application was filed on 17.11.2025 as a divisional application to the application mentioned under INID code 62.

(54) **DEGRADABLE ELASTOMERIC MATRICES, COMPOSITIONS COMPRISING THEM, METHODS OF PREPARING THEM AND USES THEREOF**

(57) Provided herein is an elastomeric matrix, comprising poly(vinyl alcohol) (PVOH); water, and one or more plasticizers. A combined mass ratio of the one or more plasticizers to said PVOH is at least 2: 1.

Figure 3

**(Cont. next page)**

(52) Cooperative Patent Classification (CPC): (Cont.)

C-Sets
**C08L 29/04, C08L 29/04;**
**C08L 29/04, C08L 29/04, C08K 5/053;**
**C08L 29/04, C08L 29/04, C08L 71/02**

**Description**

RELATED APPLICATION/S

This application claims the benefit of priority of U.S. Provisional Patent Application Nos. 63/462,269, filed on April 27, 2023 and 63/463,034, filed on April 30, 2023, the contents of which are incorporated herein by reference in their entirety.

FIELD AND BACKGROUND OF THE INVENTION

The present invention, in some embodiments thereof, relates to polyvinyl alcohol (PVOH) based elastomeric matrix and uses thereof and, more particularly, but not exclusively, to degradable PVOH based elastomeric matrix and uses thereof.

U.S. Patent No. 4,874,562 discloses a method of molding a polyvinyl alcohol contact lens.

U.S. Patent No. 4,663,358 discloses a porous and transparent hydrated gel that is prepared from a poly(vinyl alcohol) solution in a mixed solvent consisting of water and a water-miscible organic solvent.

U.S. Patent No. 10,513,588 discloses water-soluble polyvinyl alcohol film with plasticizer blend.

International Patent Application No. WO2022/016268 disclosing biocompatible polyvinyl alcohol (PVA) matrices, composed of blends of differently hydrolyzed PVAs.

U.S. Patent Application Publication No. US20220168142A1 disclosing implantable bioerodible inserts for delivering an active pharmaceutical ingredient to the eye. The invention also relates to methods of treatment using such inserts as well as methods of manufacturing such inserts.

U.S. Patent Application Publication No. US20170226298A1 disclosing water-soluble films including a polyvinyl alcohol (PVOH) resin blend and optionally one or more additional components such as plasticizers, fillers, surfactants, and other additives.

SUMMARY OF THE INVENTION

Following is a non-exclusive list including some examples of embodiments of the invention. The invention also includes embodiments which include fewer than all the features in an example and embodiments using features from multiple examples, whether expressly listed below or not.

Example 1. An elastomeric matrix, comprising:

　　a. poly(vinyl alcohol) (PVOH);
　　b. one or more organic plasticizers; where a combined mass ratio of said one or more organic plasticizers to said PVOH is at least 2:1; and
　　c. water;

wherein said PVOH comprises PVOH of two or more types that differ from one another in one or both of hydrolysis degree (HD) and chain length.

Example 2. The elastomeric matrix according to example 1, wherein said PVOH comprises PVOH of two or more types that differ from one another in hydrolysis degree (HD).

Example 3. The elastomeric matrix according to example 1 or example 2, wherein two types of the PVOH differ in chain length by at least 1000, and have similar degrees of hydrolysis, both between 97% and 100%.

Example 4. The elastomeric matrix according to any one of examples 1-3, wherein a first type of said two or more types has a hydrolysis degree from 97 % to 100 %.

Example 5. The elastomeric matrix according to any one of examples 1-4, wherein a second type of said two or more types has a hydrolysis degree is lower than 93%.

Example 6. The elastomeric matrix according to any one of examples 1-5, wherein a second type of said two or more types has a hydrolysis degree from 80 % to 93%.

Example 7. The elastomeric matrix according to any one of examples 1-6, wherein a first type of said two or more types has chains with more than 2500 units.

Example 8. The elastomeric matrix according to any one of examples 1-7, wherein a second type of said two or more types has chains with less than 1500 units.

Example 9. The elastomeric matrix according to any one of examples 1-8, wherein a relation between a first type of PVOH and a second type of PVOH of said two or more types is from about 3:1 to about 1:3.

Example 10. The elastomeric matrix according to any one of examples 1-9, wherein a first type of said two or more types has chains with more than 2500 units and hydrolysis degree of 97% to 100%, and a second type of said two or more types has chains with less than 1000 units and hydrolysis degree of 80% to 93%.

Example 11. The elastomeric matrix according to example 3, wherein said second type of PVOH makes more than 50% of said PVOH.

Example 12. The elastomeric matrix according to any one of examples 1-11, wherein said at least two types of PVOH determine a time of degradation of said elastomeric matrix.

Example 13. The elastomeric matrix according to example 12, wherein said degradation comprises change in mechanical properties under wet conditions.

Example 14. The elastomeric matrix according to example 12, wherein said degradation comprises change in shape under wet conditions.

Example 15. The elastomeric matrix according to any one of examples 1-14, wherein said at least two types of PVOH determine a time development of mechanical properties of said elastomeric matrix under wet conditions.

Example 16. The elastomeric matrix according to any one of examples 1-15, wherein a combined mass content of said PVOH and said one or more organic plasticizers is at least 70 %wt of the total weight of the matrix excluding said water.

Example 17. The elastomeric matrix according to any one of examples 1-16, wherein said elastomeric matrix is used as an ophthalmic device.

Example 18. The elastomeric matrix according to any one of examples 1-17, wherein said combined mass ratio of said one or more organic plasticizers to said PVOH is less than 20:1.

Example 19. The elastomeric matrix according to any one of examples 1-18, characterized by substantially isotropic swelling and shrinking.

Example 20. The elastomeric matrix according to any one of examples 1-19, wherein said elastomeric matrix swells by less than 50 % by volume under wet conditions.

Example 21. The elastomeric matrix according to any one of examples 1-20, wherein said one or more organic plasticizers is independently selected from the group consisting of a polyol, a polyprotic organic acid, a polyamine, an alkyl gluceth, an aliphatic polyalkylene glycol, an ethanolamine, a saccharide, an oligosaccharide, an amino acid, a polyphenol, tromethamine, urea, tannic acid, and any salt thereof and/or combinations thereof.

Example 22. The elastomeric matrix according to example 21, wherein said polyol is selected from the group consisting of ethylene glycol, diethylene glycol, triethylene glycol, tetraethylene glycol, propylene glycol, triacetin erythritol, a poly-glycol, a poloxamer, and a copolymer thereof, and glycerol and esters thereof.

Example 23. The elastomeric matrix according to example 21, wherein said polyprotic organic acid is selected from the group consisting of oxalic acid, maleic acid, citric acid, and any salt thereof.

Example 24. The elastomeric matrix according to example 21, wherein said polyamine is selected from the group consisting of spermine, spermidine, diethylenetriamine, triethylenetetramine, tris(2-aminoethyl)amine, a polyethylenimine, and any salt thereof.

Example 25. The elastomeric matrix according to example 21, wherein said aliphatic polyalkylene glycol is selected from the group consisting of a polyethylene glycol, a polypropylene glycol, a poly-glycol, a poloxamer, and a polysorbate.

Example 26. The elastomeric matrix according to example 21, wherein said aliphatic polyalkylene glycol is polyethylene glycol.

Example 27. The elastomeric matrix according to examples 24-25, wherein a mass content of said PVOH is substantially equal to a mass content of said aliphatic polyalkylene glycol.

Example 28. The elastomeric matrix according to example 27, wherein said aliphatic polyalkylene glycol is selected from the group consisting of polyethylene glycol, polypropylene glycol, and any mixture thereof.

Example 29. The elastomeric matrix according to any one of examples 1-28, wherein said water constitute less than 50 %wt of the total mass content of the matrix.

Example 30. The elastomeric matrix according to any one of examples 1-29, wherein said mass content of said PVOH is less than 25 %wt of the total weight of non-water ingredients of the matrix.

Example 31. The elastomeric matrix according to any one of examples 1-30, wherein said mass content of said PVOH is less than a third of the total weight of said one or more organic plasticizers.

Example 32. The elastomeric matrix according to any one of examples 1-31, essentially devoid of covalent crosslinking.

Example 33. The elastomeric matrix according to any one of examples 1-32, further comprising a pharmaceutically active agent.

Example 34. An ophthalmic device comprising the elastomeric matrix according to example 1.

Example 35. The ophthalmic device according to example 34, wherein said elastomeric matrix consists of ophthalmically acceptable ingredients.

Example 36. The ophthalmic device according to example 34 or example 35, wherein said device weights between 3 mg and 50 mg.

Example 37. The ophthalmic device according to any one of examples 34-36, wherein said elastomeric matrix comprises a pharmaceutically active agent.

Example 38. The ophthalmic device according to any one of examples 34-37, wherein said ophthalmic device is configured to be positioned on a surface of an eye.

Example 39. The ophthalmic device according to example 38, wherein said surface is at least partially underneath at least one of the upper eyelid, the lower eyelid and outside a cornea of the eye.

Example 40. The ophthalmic device according to any one of examples 34-39, wherein said ophthalmic device is configured for delivering at least one pharmaceutically active agent to an eye for an extended period of time of between 5 minutes and 24 hours.

Example 41. A medical device comprising two elastomeric matrices, each according to example 1, wherein the two matrices differ from one another in one or more of:

a. ratio between the mass content of PVOH included in the matrix and the mass content of the matrix;
b. ratio between the mass content of PVOH included in the matrix and the combined mass contents of the one or more plasticizers;
c. the types of PVOH; and
d. mass ratios between the types of PVOH.

Example 42. The medical device according to example 41, wherein the two matrices touch across a touching surface.

Example 43. The medical device according to example 42, wherein the touching surface is a closed surface.

Example 44. The medical device according to any one of examples 41-43, wherein the two matrices degrade under wet conditions at different rates.

Example 45. The medical device according to any one of examples 41-44, wherein the two matrices show distinct mechanical properties under dry conditions.

Example 46. An elastomeric matrix, comprising:

a. poly(vinyl alcohol) (PVOH);
b. one or more organic plasticizers; where a combined mass ratio of said one or more organic plasticizers to said PVOH is at least 2:1; and
c. water;

wherein said PVOH comprises only PVOH of chains shorter than 2000 units or shorter than 1000 units and degree of hydrolysis smaller than 90%, and
said water makes less than 10% or less than 5% of the matrix.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

Figure 1 is a graph illustrating exemplary degradation characteristics of three different elastomeric matrix compositions, as they evolve in time under wet conditions, according to some embodiments of the invention;
Figure 2 is a graph illustrating degradation of some matrices during wetting and drying cycle, according to some embodiments of the invention;
Figure 3 is a simplified schematic cross sectional view of a medical device, according to some embodiments of the invention;
Figures 4a-b are simplified schematic cross sectional views of medical device, according to some embodiments of the invention;

Figures 5, 6, 7, 8, 9 and 10 are simplified schematic cross sectional views of portions of ophthalmic devices, according to some embodiments of the invention; and

Figures 11a-b are simplified schematic cross sectional views of a device on an eye surface, according to some embodiments of the invention.

## DESCRIPTION OF SPECIFIC EMBODIMENTS OF THE INVENTION

The present invention, in some embodiments thereof, relates to polyvinyl alcohol (PVOH) based elastomeric matrix and uses thereof and, more particularly, but not exclusively, to degradable or biodegradable or bioerodible PVOH based elastomeric matrixes. The terms "biodegradable" and "bioerodible" (as well as their inflictions, such as biodegradability and bioerodibility) are used herein interchangeably. For example, a matrix may be considered biodegradable if it erodes mechanically in the body, whether or not any of its constituents is chemically decomposed.

## Overview

An aspect of some embodiments of the invention relates to elastomeric matrices (referred hereinafter just as "matrix" or "matrices/matrixes"), which comprise one or more types of PVOH in order to achieve a matrix having determined and/or desired mechanical properties. In some embodiments, the elastomeric matrices are PVOH-based, in the sense that PVOH is the sole or main film forming polymer in the matrix.

In some embodiments, a matrix is designed to dissolve and/or degrade under wet conditions according to predetermined timeline requirements. In some embodiments, the timeline of degradation is controlled by choosing a composition of the one more types of PVOH in the matrix. In some embodiments, the composition is optionally characterized by the various types of PVOH included in the composition and their relative masses, or the mass portion of the total PVOH provided by each of the types.

In some embodiments, when referring to "wet conditions", it should be understood to relate to conditions wherein a matrix of the invention is in direct contact with liquid (immersed in the liquid or after immersion in the liquid and before the liquid evaporated or wiped away). In some embodiments, the liquid is a naturally occurring bodily fluid, including but not limited to blood, saliva, tears, excreta, body tissue, tissue fluids), an aqueous solution (e.g., of one or more organic plasticizers), a buffer solution, and any combinations thereof. In some embodiments, the liquid is naturally occurring or simulating a naturally occurring fluid, such as artificial tear fluid. In some embodiments, artificial tear fluid is based on an aqueous solution containing about 0.67 % sodium chloride, about 0.2 % sodium bicarbonate and about 0.008 % calcium chloride. In some embodiments, "wet conditions" refer to the conditions when the matrix is positioned on mucous tissue of a human, for example, on buccal tissue or an eye surface.

In some embodiments, the term degradation of a matrix is defined as the change in properties of the matrix over time under wet conditions, and "degradability" as the tendency to degrade or the rate of degradation.

In some embodiments, the change in properties may include, for example, a change or loss in mass of one or more of the matrix components or of the matrix as a whole. For example, while plasticizers that leave the ophthalmic device may be replaced by water, in some embodiments, PVOH itself leaves the ophthalmic device, and thus, the device loses weight. Alternatively, or additionally, the change may include change in mechanical properties, shape, and/or size of the matrix. Alternatively, or additionally, the degradation may be expressed as dissolution, breakage, tear, and/or crumbling of the matrix. In general, the degradation may be due to breakage of bonds between the different constituents of the matrix (e.g., PVOH, plasticizers, and water) while each of the constituents stays intact. In some embodiments, for example, when the matrix is used for medical purposes, the degradation allows washing the ophthalmic device, or the elastomeric matrix it comprises, out of the body. For example, when used in ophthalmic devices, the degradation of the matrix may allow washing the remains of the matrix out of the eye by tears.

In some embodiments, the kind of degradation, e.g., the change from soft solid to slime, dissolution, mass loss, or change in tearability, may depend on the types of PVOH included in the matrix. For example, the inventors found that some matrices that include long chain fully hydrolyzed (LCFH) PVOH and short chain partially hydrolyzed (SCPH) PVOH tend to become "slimy" when immersed in simulated tear fluid (STF). In such embodiments, the increased sliminess may be accompanied by a decrease in compression modulus with time under STF. In some embodiments, some matrices that include LCFH PVOH and long chain partially hydrolyzed (LCPH) PVOH tend to become more easily dissolvable in STF, in the sense that they may dissolve to clear solution in cold water. In some embodiments, some matrices that include LCFH PVOH and short chain fully hydrolyzed (SCFH) PVOH tend to be torn more easily the longer they are immersed in STF. In some embodiments, increased susceptibility to tearing of such matrices may be accompanied by decrease in compression strength with time under STF.

See also Exemplary Mechanical Properties in Example 7 below.

In some embodiments, the degradation may obviate the need to take the matrix out of the body. For example, the products of the degradation may be expelled from the body naturally, e.g., by tear fluid (if the matrix is used in the eye) by urea (if the matrix is used in the bladder), etc. In case of ophthalmic devices, after some time in the eye, an ophthalmic device made of a degradable matrix may leave the eye naturally, with no need to take it out actively from the eye. For example, the device may fully dissolve and leave the eye with tear fluid, or disintegrate to such small parts that may be expelled from the eye as any other foreign object.

In some embodiments, slightly pressing a degradable matrix (e.g., used as ophthalmic device) may bring to immediate change in the form or integrity of the matrix, so an ophthalmic device may be naturally expelled shortly after being pressed.

In some embodiments, the degradation causes the matrix to acquire a shape and/or consistency that are comfortable to the anatomic locale at which it is used, so it may remain there. For example, an ophthalmic device may acquire a shape conforming to the space between the ocular and tarsal conjunctivae, where it may rest without discomforting the patient until it is degraded and expelled from the eye. In some embodiments, on the way out from the eye a degraded device might come across the cornea and cause temporary discomfort.

In some embodiments, the terms "dissolve" and "degrade" are interchangeable and refer to the process by which the elastomeric matrices change, e.g., under wet conditions. The change may be, for example, from a solid state to a completely degraded state. The change may be, for example, loss in mass. In some embodiments, a completely degraded state can be defined, for example, as a degradation state of the matrix when the matrix cannot be seen by the naked eye, for example because the matrix has disintegrated to particles of very small size, and/or, for example, a state of the matrix when the matrix elastically changes shape and/or does not hold a defined form under certain external pressures, for example, in matrices used in or applied to any tissue, for example on the eye, pressure from the eyelid on the matrix. The terms "shape" and "geometry" are interchangeable and refer to the form of the matrix.

In some embodiments, the matrixes comprise a mixture of two or more types of PVOH. In some embodiments, the differences in the types of PVOH are in one or more of hydrolysis degree and length of PVOH chains. In some embodiments, the higher the quantity of highly hydrolyzed PVOH in the matrix, the longer the time that will take for the matrix to fully degrade. In some embodiments, said PVOH comprises PVOH of two or more types that differ from one another in one or both of hydrolysis degree (HD) and chain length. In some embodiments, each of said two or more types of PVOH constitutes at least 10% of the total amount of PVOH in the matrix. In some embodiments, the two or more types of PVOH include one type that has long chains and is fully hydrolyzed, and one type that has at least one of: short chains and is partially hydrolyzed; long chains and is partially hydrolyzed; short chains and is fully hydrolyzed. In some embodiments, short chains are of 200 to 2000 monomeric units; long chains are of 2200 to 5000 monomeric units; fully hydrolyzed PVOH is of hydrolysis degree of 97% or more; and partially hydrolyzed is of hydrolysis degree of 95% or less.

In some embodiments, the number of PVOH types is three or more.

In some embodiments, two of the types of PVOH differ in chain length, e.g., by at least 1000 or 1500 units. In some such embodiments, the two of the types of PVOH have similar degrees of hydrolysis, e.g., between 97% and 100%.

In some embodiments, two of the types of PVOH differ in degree of hydrolysis, e.g., one has D.H of 97% or more, and the other of 93%, 90%, or less. In some such embodiments, the two types of PVOH differ also in chain length, e.g., by at least 1000 or 1500 units. For example, the PVOH type with the higher degree of hydrolysis may have longer chains. In another example, the PVOH type with the higher degree of hydrolysis may have shorter chains. In some embodiments, in which two of the types of PVOH differ in degree of hydrolysis, the two types of PVOH have similar lengths, differing from each other by, e.g., less than 500 units.

In some embodiments, the PVOH with short chains has an average chain length of between about 200 and about 2000, monomeric units; and the PVOH with long chains has average chain length between about 2200 and about 5000 monomeric units. In some embodiments, the fully hydrolyzed PVOH has a hydrolysis degree of 97% or more; and the partially hydrolyzed PVOH has hydrolysis degree of 94% or less. In some embodiments, short chains have a molecular weight lower than about 50 Kg/Mol to about 80Kg/Mol, while long chains have a molecular weight higher than about 50 Kg/Mol to about 80Kg/Mol.

In some embodiments, the matrices are specifically designed to keep their geometry/shape under wet conditions, before the initiation of the degradation process. The degradation process may begin, in some embodiments, after 1 minute under wet conditions and in other embodiments after several hours under wet conditions, or within intermediate times. A matrix that keeps its geometry may be shaped outside the body (e.g., the eye), knowing that the same shape will be retained at least for some period after contacting the body.

In some embodiments, the elastomeric matrix comprises PVOH, one or more organic plasticizers, and optionally water. In some embodiments, the elastomeric matrix is characterized by a ratio between the combined mass of the one or more plasticizers to a combined mass of the PVOH of the various types that is at least 2:1. That is,

$$\frac{total\ mass\ of\ plasticizer(s)}{total\ mass\ of\ PVOH} \geq \frac{2}{1}.$$

This ratio is referred to herein as "combined mass ratio".

In some embodiments, the elastomeric matrix comprises more than one type of plasticizer.

In some embodiments, the matrix is characterized by keeping a geometry when coming in contact with a wet environment, for example when put in contact with the eye or other body tissue. In some embodiments, in addition to keeping its geometry when coming in contact with a wet environment, optionally, the matrix increases its volume. In some embodiments, the volume increase is by no more than 50%. In some embodiments, the swelling is the same in all directions, so that the volume changes, but the geometry is kept. In some embodiments, the elastomeric matrix is characterized by substantially isotropic swelling when being immersed in simulated tear fluid and at room temperature for 5, 10, or 15 minutes. In some embodiments, the elastomeric matrix swells by less than 50 % by volume when immersed in simulated tear fluid for 5, 10, or 15 minutes at room temperature.

In some embodiments, the mechanical properties and/or the degradability of the elastomeric matrix depends on the types of PVOH used to form the matrix, and their respective masses. For example, when the PVOH includes mainly long chain fully hydrolyzed (hereinafter LCFH) PVOH and a minority of short chain partially hydrolyzed (hereinafter SCPH) PVOH the matrix degrades more slowly than when the LCFH PVOH is the minority of the PVOH in the matrix and SCPH PVOH is the majority thereof.

The following table (Table 1) provides examples of some commercially available types of PVOH. The list is taken from the brand Mowiol™, but similar materials are available from other sources. The PVOH type named "30k" in the table below (Table 1) is not of the Mowiol brand. All PVOH types used in the examples and measurements recited herein were carried out with PVOH sold under the brand of Emprove™ or with the 30k PVOH (which is Sigma Aldrich 8.21039).

TABLE 1

|  |  | Name | MW [kg/mol] | Chain length | Hydrolysis degree [%] |
|---|---|---|---|---|---|
| Partially hydrolyzed | Short chain (SCPH) | 3-83 | 14 | 270 | 83 |
|  |  | 4-88 | 31 | 630 | 88 |
|  |  | 5-88 | 37 | 750 | 88 |
|  |  | 8-88 | 67 | 1400 | 88 |
|  | Long chain (LCPH) | 18-88 | 130 | 2700 | 88 |
|  |  | 23-88 | 150 | 3100 | 88 |
|  |  | 26-88 | 160 | 3300 | 88 |
|  |  | 40-88 | 205 | 4200 | 88 |
|  |  | 30-92 | 175 | 3700 | 92 |
| Fully hydrolyzed | Short chain (SCFH) | 3-98 | 16 | 360 | 98 |
|  |  | 4-98 | 27 | 600 | 98 |
|  |  | 6-98 | 47 | 1000 | 98 |
|  |  | 10-98 | 61 | 1400 | 98 |
|  |  | 30k | 30 | 670 | 99 |
|  | Long chain (LCFH) | 20-98 | 125 | 2800 | 98 |
|  |  | 56-98 | 195 | 4300 | 98 |
|  |  | 28-99 | 145 | 3300 | 99 |

In some embodiments, the elastomeric matrix is used as a medical device. For example, the matrix may be used as an ophthalmic device or make part of an ophthalmic device. In some embodiments, the ophthalmic device may be configured to be positioned on the surface of the eye, preferably on the sclera optionally without covering any portion of the cornea so as not to hinder sight. In some embodiments, options of positioning the ophthalmic device include one or more of: topical (sclera, cornea), subconjunctiva, conjunctiva, suprachoroidal, intraviteral, intra cameral, subretinal, or any other way to provide to the eye. In some embodiments, the medical device is applied onto or into body parts other than the eye, for example, body cavities, (e.g., bladder, stomach) joints, etc. In some embodiments, the matrix may be applied to a body part by intramuscular injection. In some embodiments, a potential advantage of positioning on the sclera is that the sclera is potentially less sensitive (to pain, irritation, etc.) than the cornea, so designing the matrix to contact only the sclera potentially results in a matrix that is more convenient for the user. In some embodiments, depending on the composition of the PVOH types included in the elastomeric matrix, complete degradation occurs, for example, between 0.5 hours and 12 hours from contacting the eye. Optionally from between 0.5 hours and 24 hours. Optionally between 0.5 hours and 48 hours. Optionally within longer than 48 hours. In some embodiments, a potential advantage of controlling the degradation time is that it allows a user to use the ophthalmic device during convenient and/or chosen hours (for example: while the user is sleeping, or for example to allow the device to act at a desired time window).

In some embodiments, the elastomeric matrix is characterized under dry conditions by a tensile strength below 10, 4, 3, MPa, preferably below 2 MPa. Additionally or alternatively, the elastomeric matrix is characterized under dry conditions by elasticity (aka Young Module) of 0.01 MPa to 10, 4, or 3 MPa, preferably 0.01MPa to 2MPa. Additionally or alternatively, the elastomeric matrix is characterized under dry conditions by an elongation at breakage of at least 50%, for example, 50% to 900% or 50% to 1000%. In some embodiments, a potential advantage to have a matrix characterized the above-mentioned mechanical properties is that it potentially reduces discomfort in the user, thereby potentially increasing compliance.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Exemplary basic composition of an exemplary elastomeric matrix

In some embodiments, an exemplary elastomeric matrix for use, for example but not limited to, as an ophthalmic device, comprises poly(vinyl alcohol) (PVOH), one or more, two or more, or three or more plasticizers, none of which is water, and water. In some embodiments, each of the one or more, two or more, or three or more plasticizers is an organic plasticizer. In some embodiments, the combined mass ratio (i.e., the ratio between the combined mass of the one or more plasticizers to the combined mass of PVOH of the various types included in the matrix is at least 2:1, for example 2.5:1, 3:1, 4:1 or higher or intermediate ratios. In some embodiments, the mass content of all non-water ingredients of the matrix is at least 70 %wt of the total weight of the matrix excluding the mass of water, namely the total mass of the PVOH and the plasticizer(s) is at least 70 %wt, at least 80 %wt or at least 90 %wt of the mass of all non-water ingredients of the matrix.

In some embodiments, the mass ratio of the plasticizer(s) to PVOH is less than 5:1, less than 10:1, less than 15: 1, less than 20:1, or less than 30:1. For example 5.6:1. In some embodiments the mass ratio of the plasticizer(s) to PVOH ranges from 10-2: 1 (from 10:1 to 2:1), or 30:1 to 3:1.

In some embodiments, the mass content of PVOH (of all types combined) constitutes less than 33 %wt of the total weight of non-water ingredients of the matrix. For example, the mass content of PVOH constitutes, in some embodiments, between 3% and 10 %wt or less, between 5% and 15 %wt, less than 20 %wt, less than 25 %wt, or less than 30 %wt of the total weight of the PVOH and plasticizers together (non-water ingredients of the matrix).

The term "combined mass ratio", as used herein, refers to the combined amount of one or more ingredients of a first kind (e.g., the one or more plasticizers) relative to the amount of one or more other ingredients of the matrix (e.g., the types of PVOH), wherein all the amounts are provided in terms of mass (e.g., gram). The combined mass ratio is calculated by summing the mass of the one or more ingredients of the first kind to obtain a first combined mass; summing the mass of the one or more ingredients of the second kind to obtain a second combined mass and dividing the first combined mass by the second combined mass.

The term "combined mass", as used herein, refers to the combined amount of a plurality ingredients, provided in terms of mass. The combined mass is calculated by summing the masses of the ingredients making up the plurality.

The term "elastomeric matrix", as used herein, refers to a crosslinked polymeric structure form that displays rubber-like elasticity and can undergo deformation under the influence of a force and regain its original shape once the force has been removed. In some embodiments, the crosslinking is purely physical, i.e., the matrix is devoid of covalent crosslinking. Being devoid of covalent crosslinking may be expressed in being soluble in hot water. The exact temperature and amount of hot water to dissolve a specific matrix may depend on the size of the matrix and its specific formulation, including the types of PVOH included in the formulation. In some embodiments, the solubility of the matrix is about the solubility of the less soluble of said types of PVOH.

In some embodiments, an elastomeric matrix comprising less than 33 % by weight (%wt) of polyvinyl alcohol has the advantage of maintaining its elastomeric matrix form under dry conditions; swells by less than 50 % by volume (or less than 15 % along each of its three dimensions) under wet conditions; and substantially maintains its geometry when moving from dry to wet conditions. Manipulating these properties were found to require the use of at least two plasticizers.

In the context of some embodiments of the present invention, the elastomeric matrix is a form of physically crosslinked polymeric structure that can release one or more of its ingredients (for example plasticizers and/or active pharmaceutical ingredients (API)) to water (or any wet environment) with or without change in its mechanical properties, for example, having an open-cell porous microstructure that can incorporate sequestered and/or added releasable substances within its interconnected voids. According to some embodiments of the present invention, the elastomeric matrix is a network of hydrogen-bonded polymers and plasticizers. According to some embodiments of the present invention, the elastomeric matrix is a network of hydrogen-bonded polymers, plasticizers and water. According to some embodiments of the present invention, the elastomeric matrix is a network of hydrogen-bonded polymers and plasticizers. According to some embodiments of the present invention, the elastomeric matrix is a network of hydrogen-bonded polymers, plasticizers and water. In some embodiments, the elastomeric matrix provided herein is essentially devoid of covalent crosslinking. Being essentially devoid of covalent crosslinking may be expressed as being water soluble in hot water. The amount and temperature of the water required to dissolve the matrix depends on the size of the matrix and its formulation, including the specific PVOH types that the matrix includes. In some embodiments, the solubility of the matrix is about the solubility of the less soluble of said PVOH types.

In some embodiments, the elastomeric matrix provided herein has a porosity of about 50-90 %. In some embodiments, the matrix is characterized by a porosity of at least about 50 %, 60 %, 70 %, 80 % or at least about 90 %. In some embodiments, the matrix is characterized by a porosity of at least about 50 %, 60 %, 70 %, 80 % or at least about 90 %.

In some embodiments, the PVOH-based elastomeric matrix is free of chemical crosslinker residues and stable during soaking in aqueous medium in terms of geometry and consistency, at least at the first stages in the aqueous medium, for example during the first 1 to 2 minutes after insertion in the aqueous medium, or for longer times, up to, for example, several hours, depending on the formulation. In some embodiments, the elastomeric matrix is both soft and elastic under dry and wet conditions.

In some embodiments, the consistency of the elastomeric matrix provided herein may resemble that of a hydrogel, however, in distinction from hydrogels, the elastomeric matrix provided herein has low water content (less than 50 %w), is stable under dry conditions, and does not have to be immersed in water in order to retain its pliability. In some embodiments, under wet conditions, the water content may reach no more than 150% of the mass of the non-PVOH components the matrix had under dry conditions.

In some embodiments, the mechanical properties of the elastomeric matrix provided herein resemble that of rubber (elastomeric) under dry and/or wet conditions (e.g., soaked in an aqueous medium).

Unless explicitly stated otherwise, any reference to the elastomeric matrix provided herein is made to the elastomeric matrix under dry conditions. The term "dry conditions" refers to the elastomeric matrix provided herein itself, without referring to a solution that the matrix may or may not be soaked in/with.

In some embodiments, water is structurally fixed in the elastomeric matrix, is free to evaporate/flow out of the matrix, or is not present in the matrix. In some embodiments, the exact amount of water (fixed and/or free) varies depending on the nature of the ingredients in the matrix and the conditions it is kept in. In some embodiments, when referring to the "non-water" elements/ingredients of the elastomeric matrix provided herein, it is meant to refer to all its ingredients excluding water. In some embodiments, the mass content of the "non-water" elements is not to be confused with the mass content of the matrix under dry conditions, which may include a mass of water.

When referring to "dry conditions" it should be taken as relating to conditions wherein the elastomeric matrix as provided herein is exposed to air at room temperature in an open or closed container and is not immersed in liquid, such as water or any other aqueous medium or liquid.

In some embodiments, in the context of ophthalmic devices, "dry conditions" describes a state that is the opposite of how contact lenses are stored; contact lenses are typically made of a hydrogel, which should always be kept under aqueous medium in order to retain their shape and malleability. In sharp contrast, the elastomeric matrix provided herein does not have to be kept wet to retain shape and malleability; and does not change its shape (although it may change in size) when going from wet to dry conditions and back.

Hence, in some embodiments, when the elastomeric matrix is said to have less than a certain %wt polyvinyl alcohol of the total weight of the non-water ingredients of the matrix, it is meant that the mass content of PVOH constitutes less than the certain %wt of the total mass content of the matrix excluding any water, structurally fixed in the matrix (if any) or free to flow out of the matrix.

In some embodiments, a relatively small amount of water in the matrix allows it to have longer shelf-life, and to be storable under dry conditions (i.e., not soaked in a liquid), whereas excessive amount of water is not required and undesired. In some embodiments, assuming only water evaporates from the wet elastomeric matrix, the amount of water remaining after conventional drying is seen as forming a part of the matrix. In the context of the present invention, conventional drying is drying by exposing the outer surface of the matrix to ambient air under room temperature and ambient humidity. In some embodiments, the room temperature may range 15-30 °C, and the relative humidity may range 30-75 %. In some embodiments, the exact conventional drying time may vary with the specific content of the matrix, but is typically between 24 hours and four days. In some embodiments, the drying is accelerated, e.g., by heating to about 60°C, and the drying time shortens accordingly. In some embodiments, the drying is slowed down, e.g., by drying the solution in a closed mold and/or with cooling, e.g., to about 10°C.

In some embodiments, the amount of water can be determined, among other methods, by the Karl Fischer method, and the direct measurement of loss of mass upon drying, or LOD method. The Karl Fischer method was conducted volumetrically using Karl Fischer instrument model Titrando 852 (Metrohm), with Hydranal composite 5 (Honeywell) as titrant. Methanol was used as the solvent. The LOD test was conducted under a temperature of 85 °C, using MX-50 moisture analyzer by A&D Company Ltd, Japan, on samples of 700 mg or larger.

According to these methods, which give similar results for a given sample, in some embodiments of the present invention, the elastomeric matrix includes less than about 50 %wt of water therein. In some embodiments, the amount of water is less than about 40 %wt, less than about 30 %wt, less than about 25 %wt, less than about 20 %wt, less than about 15 %wt, less than about 10 %wt, or less than about 5 %wt of the total mass of the matrix. In some embodiments, the elastomeric matrix includes between about 50 %wt of water and about 5 %wt of water.

In some embodiments, due to its unique composition, the elastomeric matrix provided herein swells under wet conditions, while preserving its shape, by less than 50 % in volume. In some embodiments, the matrix swells by no more than 40 % by volume, or less than 35 %, or less than 20 %, or less than 15 %, or less than 10 %, or less than 5 % by volume under wet conditions.

It is noted herein that, in some embodiments, the matrix may be cast, molded, cut or otherwise made with a distinct 3D shape, which essentially does not change when conditions change from dry to wet and *vice versa*. This property is referred to herein as shape retention. In other words, while the overall volume of a piece of the matrix provided herein may change (swell or shrink) under changing wetting conditions, the overall shape of the piece remains essentially the same, without distortions, compression or deformations. Hence, in some embodiments, another characteristic of the elastomeric matrix provided herein is substantially uniform size variability along all directions and orientations under changing wetting conditions. Substantially uniform meaning that the size varies along each direction by the same amount ±20 %. This characteristic may also be referred to herein as isotropic swelling and shrinking. In some embodiments, the elastomeric matrix is characterized by substantially isotropic swelling when being immersed in simulated tear fluid and at room temperature for 5, 10, or 15 minutes. In some embodiments, the elastomeric matrix swells by less than 50 % by volume when immersed in simulated tear fluid for 5, 10, or 15 minutes at room temperature.

In some embodiments, the composition and method of preparing the elastomeric matrix provided herein also determine the degree of crystallinity of the matrix. Degree of crystallinity (DoC) is the fraction of the ordered molecules in a polymeric substance, which typically ranges between 10 % and 80 % for many known polymeric substances. Higher values can be achieved in materials having small molecules, or in polymeric substances prepared and/or stored at temperatures just under the melting point. Most methods of evaluating the degree of crystallinity assume a mixture of perfect crystalline and totally disordered areas; the transition areas are expected to amount to several percent. These methods include density measurement, differential scanning calorimetry (DSC), X-ray diffraction (XRD), infrared spectroscopy and nuclear magnetic resonance (NMR).

In some embodiments, the elastomeric matrix provided herein does not require cooling to below room temperature and once set and dried, does not require heating to above room temperature. In some embodiments, the elastomeric matrix contains a pharmaceutically active ingredient (API), and is obtainable without heating the API or any mixture or solution containing the API. In some embodiments, the degree of crystallinity of the matrix is lower than 70 %, lower than 60 %, lower than 50 %, lower than 40 %, lower than 30 %, or lower than 20 %. In some embodiments, the matrix does not change its shape after freezing to temperature of -18°C or lower and thawing to room temperature. In some embodiments, a potential advantage is that is potentially allows using the matrix with active pharmaceutical ingredients (API) that require deep freezing.

In some embodiments, the elastomeric matrix provided herein is highly suitable for use as an ophthalmic device in ophthalmic uses, including uses that require the matrix to be transparent and uses that do not pose such a requirement. Thus, the elastomeric matrix provided herein according to some embodiments, is characterized by relatively low transparency. The transparency of a substance refers to the optical distinctness with which an object can be seen when viewed through a film/sheet made from the substance. The transparency of an object made from the elastomeric matrix provided herein can be measured by its total transmittance, which is the ratio of transmitted light to the incident light, while accounting for influencing factors: reflection, absorption and dispersion. For example, subtracting the absorption/dispersion and reflection, the total transmittance of a sample is the incident light (100 %) minus absorption/dispersion (X %) and minus reflection (Y %); in other terms, the total transmittance = incident light - (absorption/dispersion + reflection). In some embodiments, non-transparent elastomeric matrices may be preferred, e.g., due to being easier to produce without compromising functionality and, additionally, it facilitates the manipulation by a user since it potentially allows the user to visualize better the matrix. For example, non-transparent elastomeric matrices may be obtained with PEG as a plasticizer and/or by producing the matrices at room temperature.

According to some embodiments of the present invention, the elastomeric matrix is characterized by total transmittance (transparency) through optical length of 0.5 millimeter of less than 80 %, less than 75 %, less than 70 %, less than 65 %, less than 60 %, less than 55 %, less than 50 %, less than 40 %, or less than 30 % total transmittance of light therethrough.

In some embodiments, the elastomeric matrix provided herein further comprises at least one inorganic ion and/or salts thereof, and/or at least one organic ion and/or salts thereof.

In some embodiments, the elastomeric matrix provided herein further comprises at least one buffering agent. In some embodiments, the buffering agent is tromethamine, phosphate, citrate, and any combinations thereof.

In some embodiments, the elastomeric matrix provided herein further comprises at least one surfactant. In some embodiments, the surfactant is selected from sorbitan esters (Spans), sorbitan tristearate (Tweens), poloxamers, Tritons, Betains and any combinations thereof.

In some embodiments, a matrix of the invention is substantially devoid of chemical (covalent) crosslinks. Chemical or covalent crosslinks are covalent bonds between monomers of the same polymeric chain, two (or more) polymeric chains, or between a monomer of a polymeric chain and another molecule (e.g., plasticizer). Being essentially devoid of covalent crosslinking may be expressed as being water soluble in hot water. The amount and temperature of the water required to dissolve the matrix depends on the size of the matrix and its formulation, including the specific PVOH types that the matrix includes. In some embodiments, the solubility of the matrix is about the solubility of the less soluble of those PVOH types.

Exemplary degrees of hydrolysis (D.H.), degrees of polymerization (D.P.), degradation time and combinations thereof

The inventors have found a surprising effect when combining two types of PVOH having different degrees of hydrolysis for use in an elastomeric matrix as described herein. This effect may be beneficial, for example, for medical usage of the matrix, e.g. for ophthalmic devices, or other medical applications as described herein.

According to some embodiments of the invention, at least two types of PVOH are used for the preparation of the elastomeric matrix. In some embodiments, the difference between the types of PVOH are one or more of a degree of hydrolysis and a degree of polymerization. In some embodiments, a potential advantage of combining at least two types of PVOH is that it allows to manipulate changes over time in mechanical properties of the matrix (for example, tensile strength and/or elastic module) under wet conditions. Additionally or alternatively, in some embodiments, a potential advantage of combining at least two types of PVOH is that it allows manipulating the time that takes for the elastomeric matrix to degrade in wet conditions.

In some embodiments, a first type of PVOH used for preparing the elastomeric matrix is characterized by a full degree of hydrolysis (D.H.) of from 97 % to 100 %.

In some embodiments, a second type of PVOH used for preparing the elastomeric matrix is characterized by a partially degree of hydrolysis (D.H.) of below 95 %, for example, from 80 % to 93 %. In some embodiments, the partially degree of hydrolysis of the PVOH is less than 90 %.

It should be understood to a person having skills in the art that the abovementioned percentages are average percentages, and that in each type of PVOH a degree of hydrolysis is distributed around and within the abovementioned numbers.

The degree of polymerization (D.P.) of a polymer is estimated by dividing the molecular weight of the polymer by the molecular weight of the monomer unit.

In some embodiments, a first type of PVOH used for preparing the elastomeric matrix is characterized by a degree of polymerization comprising long chains that include chains with more than 2500 units and/or molecular weight of more than 100kg.

In some embodiments, a second type of PVOH used for preparing the elastomeric matrix is characterized by a degree of polymerization comprising shorter chains, that include, e.g., chains with less than 1500 units or less or 1000 units or less.

It should be understood to a person having skills in the art that the abovementioned percentages are average percentages, and that in each type of PVOH a degree of polymerization is distributed around and within the abovementioned numbers.

In some embodiments, molecular weight scales with chain length, but the scaling depends partially on the degree of hydrolysis, because unless the degree of hydrolysis is 100%, not all the repeating units are the same, and the molecular weight of a hydrolyzed unit is different from that of a non-hydrolyzed one. For example, for fully hydrolyzed PVOH: chain length$\cong$3.8+22.3 MW[kg], while for partially hydrolyzed PVOH: chain length$\cong$3.6+20.6 MW[kg].

In some embodiments of the present invention, the PVOH that is used for preparing the elastomeric matrix provided herein is further characterized by a degree of polymerization of that ranges 500-5000. In some embodiments, the PVOH is a mix of different degrees of polymerization, for example, it may contain one PVOH with high molecular weight (e.g., chain length of 3000 monomers or more) and one PVOH of low molecular weight (e.g., of chain length of 1000 monomer or less).

In some embodiments, the elastomeric matrix provided herein is a biodegradable matrix. In some embodiments, the elastomeric matrix is stable in liquid at room temperature more than 1 month. In some embodiments said elastomeric matrix undergoes, at least partially and gradually, dissolution and/or erosion when in contact with liquid (water, bodily fluids). In some embodiments, the elastomeric matrix provided herein is bioerodible, namely the amount of polymer bulk in the matrix decreases, due to either physical, e.g., dissolution, and/or chemical processes occurring when the matrix is in contact with bodily tissues and/or bodily liquids. In some embodiments, said bodily liquid is tear fluid.

In some embodiments, the quantities of the different types of PVOH in an elastomeric matrix correlate with the time that takes the elastomeric matrix to degrade. For example, when a ratio between fully hydrolyzed PVOH and partially hydrolyzed PVOH increases, so is the time under wet conditions to full dissolution of the matrix.

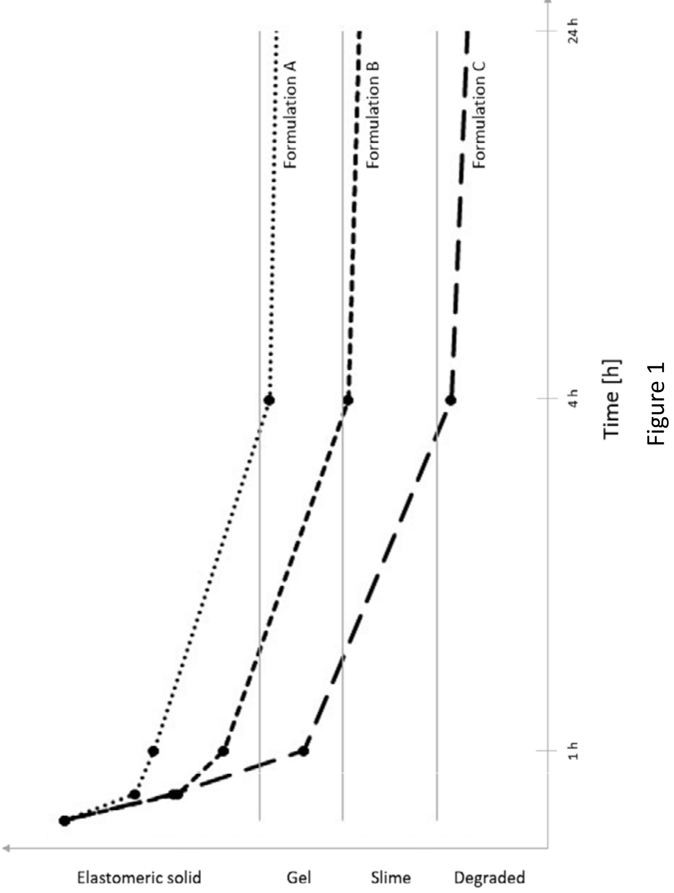

Referring now to Figure 1, showing a graph illustrating exemplary degradation characteristics of three different elastomeric matrix compositions, as they evolve in time under wet conditions, according to some embodiments of the invention. The graph shows degradation characteristics of three matrices that differ from one another only in the amounts and types of PVOH included in their compositions.

The X axis is a time axis. Matrices with various compositions may behave qualitatively similar to the behavior illustrated in the graph. One difference that may exist between behaviors of different matrices is the time duration, in which they move from one phase to another.

The Y axis is a phase axis. The values listed (elastomeric solid, gel, slime, and degraded) are qualitative descriptions of the matrix. The points are measured values of compressive modulus, presented on a logarithmic scale.

The upper (dotted) line illustrates characteristics of a first elastomeric matrix, in which all the PVOH is fully hydrolyzed and has long chains, the middle line (short dashes) illustrates characteristics of a second elastomeric matrix, in which about half the PVOH is fully hydrolyzed and has long chains, and the rest is partially hydrolyzed, and the lower (long dash) line illustrates characteristics of a third elastomeric matrix, in which about two thirds of the PVOH are partially hydrolyzed and has short chains and the rest of the PVOH is fully hydrolyzed.

The upper line shows that the first matrix stays mainly elastomeric, and may gain some gel-like characteristics after 4 hours in liquid. In some embodiments, these characteristics of gelling continue further, as illustrated by the gentle downward slope of the line towards 24 hours. It should be mentioned that some elastomeric matrices comprising only fully hydrolyzed long chains (not shown) do not show any signs of gelling after 48 hours, a week, or even three months.

The middle (dashed) line shows that the second matrix transitions from an elastomeric state to a gel-like state after about an hour and a half under wet conditions. In the gel-like phase the matrix retains the shape it had in the elastomeric phase, but becomes much more pliable, which may be expressed in lower values of elastic modulus of tension and/or elastic modulus of compression. In some embodiments, the gel-like phase may be characterized in smaller elongation at break and/or smaller tensile force than the matrix in the elastomeric phase.

The second matrix transfers into a slime phase when kept under wet conditions for about four hours. In slime phase, the matrix may have a catarrhal consistency, which may be expressed in complete collapse under application of even a slight compression force and/or breakage under application of even a slight tension force.

The third matrix (lowest line) is shown in the figure to go through the same phases in different times: the transition from elastomeric phase to gel-like phase takes place sooner than in each of the other matrices, and the transition into the slime phase takes place before the similar transition happens with the second matrix. The third matrix, however, also enters a fully degraded phase, in which the matrix does not show any measurable rejection to compression. Thus, figure 1 illustrates that in some embodiments, an elastomeric matrix composition having long chain fully hydrolyzed PVOH and short chain and/or partially hydrolyzed PVOH - the higher the quantity of short chain partially hydrolyzed PVOH the faster is the degradation process under wet conditions.

It is noted that the transition between the various phases is not necessarily sharp, and the matrix may be in numerous intermediate phases, for example, from being elastomeric to being gel-like or from being gel-like to being slime. It is also noted that not all matrices go through all the phases. While it is preferable that each elastomeric matrix is in the elastomeric phase at least under dry conditions, not all matrices go through gel-like phase, and not all the matrices go through the slime phase. It is assumed that all the matrices go to complete degradation, although not every matrix was followed in the lab for long enough to confirm that. Such details of what exact phases the matrix go through and at what time periods may depend, for example, on the exact types of PVOH used, as well as on the total amount of PVOH (e.g., as part of the total dry material in the matrix) and other ingredients of the composition and their amounts (e.g., the plasticizers).

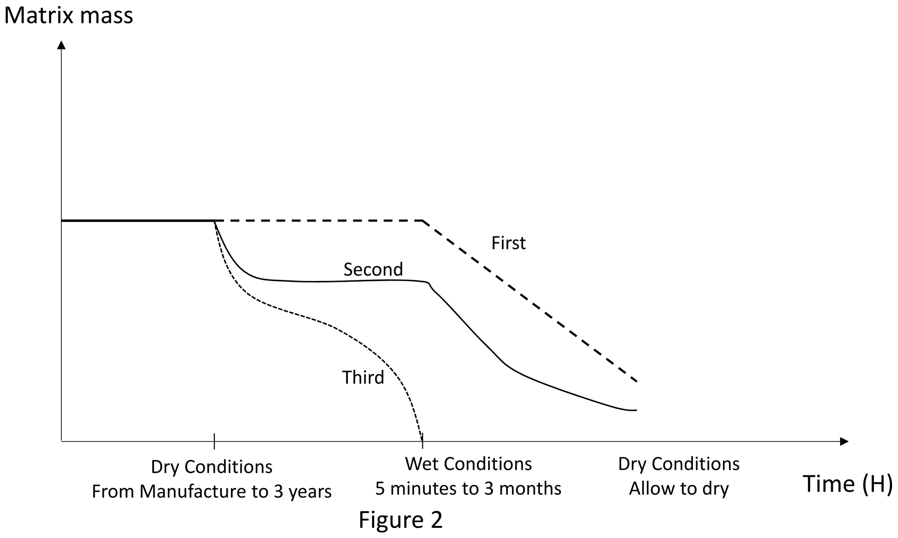

Referring now to Figure 2 showing a graph illustrating degradation of some matrices during wetting and drying cycle, according to some embodiments of the invention. The degradation is represented in the graph as the change in the mass of the matrix. This change in mass may be accompanied with other changes (e.g., in mechanical properties and/or chemical composition).

The first phase of the cycle, which may be referred to as packaged stage, goes from production of the matrix for as long as it is kept under dry conditions, possibly to the end of its shelf life. In some embodiments, the package stage may last for 3 months, 6 months, a year, two years, or even 3 years or more.

In the packaged stage, each of the matrices, illustrated by the graph, retains its respective mass. The three matrices referred to in the graph all begin with the same initial mass, and remain at the same mass for the entire first phase. Hence, the lines representing them are indistinguishable from one another. In some matrices according to embodiments of the present invention, not illustrated in the graph, some mass loss may occur during the first phase, but in most embodiments such change is not larger than 25%.

In some embodiments, during the first phase, other characteristics of the matrices also do not change, or change only nominally. These characteristics may include, for example, shape, size, chemical composition, and mechanical properties.

The second phase of the cycle, which may be referred to as a wetting stage, goes on for as long as the matrix is under wet conditions. This may last, for example, from about 5 minutes to an hour, a day, a week, a month, or even three months. In case the matrix is used for medical application, the behavior at the second phase may represent the behavior of the matrix when in contact with body fluid.

In the wetting stage, the three matrices behave differently.

In a matrix of the first kind, the mass changes of which are marked by a dashed line, the mass does not change much during the wetting stage. However, the chemical composition of the matrix may change substantially. For example, the organic elastomers making part of the matrix may be replaced by the liquid by which the matrix is wet (e.g., water or body fluids).

A matrix of the first kind tends to keep its shape and size after wetting, but their mechanical properties may change, for example, they may become softer and more pliable than before wetting.

In some embodiments, the PVOH of a matrix of the first kind is mainly long chained and fully hydrolyzed (although it may include two or more types of fully hydrolyzed long chain PVOH). In some embodiments, some of the PVOH in a matrix of the first kind may have shorter length and/or lower degree of hydrolysis, but these should preferably be in small amounts, e.g., 10% or less of the total PVOH content.

Still in the wetting stage, the degradation behavior of a matrix of a second kind is shown in the figure by a solid line. A matrix of the second kind loses a substantial portion of its weight during the wetting stage, but maintains most of its weight, no matter how long is the wetting stage. The chemical composition of such a matrix may change by the wetting both by replacement of plasticizers by water (similarly to the composition change going on a matrix of the first kind), and may also lose some of its PVOH to dissolve or disperse in the liquid. The mechanical properties of a matrix of the second kind change during the wetting stage, and the matrix becomes softer and more pliable. In some embodiments, a matrix of the second type loses some of its size during wetting stage, but remains intact.

The chemical composition of a matrix of the second kind includes a substantial part (e.g., at least 50%) of long-chain highly hydrolyzed PVOH, and the rest of the PVOH (e.g., no more than 50%) is of short chains and/or partial hydrolysis degree. It is noted that the transition between being a matrix composition of the first or second kind is not sharp, and the question if a certain composition of PVOH of different types will result in a matrix of the first or second kind may depend on the specific types of PVOH used, as well as on the kinds and amounts of the plasticizers.

Still in the wetting stage, the degradation behavior of a matrix of a third kind is shown in the figure by a dotted line. A matrix of the third kind loses nearly all its mass when under wet conditions for long enough, and loses size and shape to the extent that if not taken out of the water soon enough, it becomes shapeless and slimy. The chemical composition of such a matrix may change by losing plasticizers and PVOH. The mechanical properties of a matrix of the third kind change dramatically during the wetting stage, and the matrix may completely dissolve during the wetting stage.

The chemical composition of a matrix of the third kind includes mainly short-chain and/or partially hydrolyzed PVOH, and only small amounts of the PVOH (e.g., less than 40%) is of long chains and/or full hydrolysis degree. It is noted that the composition change required to go from a matrix of the second kind to a matrix of the third kind is not the same for all the compositions of matrices of the second kind, and the question if a certain composition of PVOH of different types will result in a matrix of the second or third kind may depend on the specific types of PVOH used as well as on the plasticizers used and their amounts.

The third phase of the cycle, which may be referred to as a drying stage, starts when the matrix is taken out of the liquid, goes on for as long as the dry conditions are maintained. In case the matrix is used for medical application, the behavior at the third phase may represent the behavior of the matrix after body fluids drained or dried (e.g., after bleeding, sweating, or tearing stops or significantly decreases).

In the drying stage, a matrix of the first kind may lose a substantial part of its mass (e.g., up to 70%), mainly because of water evaporation or water release. Shape may be kept, and size may be reduced. Re-wetting may cause the dried matrix to regain shape and size, at least partially. Flexibility and pliability are lost during drying, but may be partially regained by re-wetting.

A matrix of the second kind, goes through similar processes during the drying stage, but may lose larger part of its mass (e.g., up to 85%). Changes in size and shape will be similar to those going on matrices of the first kind, but larger. In some embodiments, a matrix of the second kind does not regain flexibility and pliability upon re-wetting.

A matrix of the third kind does not live long enough to reach the drying stage, as it may dissolve during the wetting stage. If the environment of a matrix of the third kind becomes dry before a substantial part of the mass of the matrix is dissolved, some regaining of shape and size may be achieved by re-wetting.

In some embodiments, e.g. for medical use, the required combination of types of PVOH within the elastomeric matrix may be designed to achieve a product that keeps geometrical form in dry conditions (meaning before positioning in the body). Keeping form under dry conditions potentially increases the shelf-life of the matrix, whether used as part of an ophthalmic device or as part of any other medical device. In some embodiments, keeping form under dry conditions is important since the user needs to manipulate the ophthalmic device in order to position it properly in the body of the patient, for example, in the case of ophthalmic device, on the surface of the eye. Therefore, in some embodiments, e.g., for ophthalmic devices, the combination of types of PVOH in the elastomeric matrix provides a matrix that keeps its geometry and mechanical properties in dry conditions.

In some embodiments, critical values of the combination between types of PVOH are according to the following: Where:

$$\frac{total\ PVOH}{total\ PVOH+Plasticizers} = from\ about\ 5\ \%wt\ to\ about\ 33\ \%wt$$

Matrices according to some preferred embodiments have the following PVOH compositions:

| Total PVOH | $Minimum\ \frac{LCFH\ PVOH}{Total\ PVOH}$ | $Maximum\ \frac{Short\ chain\ PVOH}{Total\ PVOH}$ |
|---|---|---|
| From about 20 %wt to about 30 %wt | 10 %wt | 90 %wt |
| From about 10 %wt to about 20 %wt | 20 %wt | 80 %wt |
| From about 5 %wt to about 10 %wt | 30 %wt | 70 %wt |

It is noted that the right column covers embodiments wherein the PVOH is fully hydrolyzed as well as embodiments wherein the PVOH is partially hydrolyzed, or a mix of fully and partially hydrolyzed PVOH.

Without being bound to theory, the degradation behavior of the various matrices according to embodiment of the present invention may be affected by the physical crosslinking, and the latter may be affected, for example, by the different types of PVOH, their relative amounts, the total amount of PVOH relative to the plasticizers or other ingredients of the matrix, the plasticizers included in the composition, and the amount of each plasticizer. Additionally, or alternatively, the degradation behavior of a matrix may be affected by chemical crosslinking (e.g., by boric acid), for example, when it is gentle enough not to interfere with the desired characteristics of the matrix.

Exemplary amounts of components within an exemplary elastomeric matrix composition

In some embodiments, an exemplary elastomeric matrix comprises one or more of the following components:

1. Water from about 5 %wt to about 50 %wt of the matrix;
2. PVOH+plasticizers from about 35 %wt to about 95 % wt of the matrix; where:

   i. The PVOH is from about 5 %wt to about 33 %wt of the PVOH+plasticizers;
   ii. The weight of the plasticizers is at least twice the weight of the PVOH

3. Miscellaneous ingredients from about 0 %wt to about 30 %wt of the weight of PVOH+plasticizers + miscellaneous ingredients.

In some embodiments, the miscellaneous ingredients may include, for example, active pharmaceutical ingredients (API), buffering agents, etc.

Exemplary mechanical properties

In some embodiments, the elastomeric matrix provided herein can substantially change its mechanical properties, such as rheological properties, elongation at break, tensile strength, yield strength, elongation at yield, elastic modulus and so forth, when exposed to wet conditions.

When referring herein to "dry conditions", it should be understood to relate to conditions wherein a matrix of the invention is exposed to air at room temperature in an open or closed container and is not immersed in liquid, and particularly not in aqueous liquid. When referring to "wet conditions", it should be understood to relate to conditions wherein a matrix of the invention is in direct contact with liquid (immersed in the liquid or after immersion in the liquid and before the liquid evaporated or wiped away).

In some embodiments, the liquid is a naturally occurring bodily fluid, including but not limited to blood, saliva, tears, excreta, body tissue, tissue fluids), an aqueous solution (e.g., of the at least one plasticizer), a buffer solution, and any combinations thereof. In some embodiments, the liquid is naturally occurring or simulating a naturally occurring fluid, such as artificial tear fluid. In some embodiments, a simulated tear fluid is based on an aqueous solution containing about 0.67 % sodium chloride, about 0.2 % sodium bicarbonate and about 0.008 % calcium chloride.

In some embodiments, the elastomeric matrix provided herein is characterized by an elongation at break of at least 50%, 100 %, 200 %, 300 %, 400 %, 500 %, 700 %, at least 800 %, or at least 900 % or at least 1000% under dry conditions. In some embodiments, the elongation at break of the matrix is at least 100 % under wet conditions (immersed in liquid or after immersion in liquid).

It was surprisingly found that in some embodiments of the present invention wherein the water content was relatively low, the elongation at break was surprisingly high. Thus, according to some embodiments of the present invention, the elastomeric matrix having a water content of 30 %wt or less, 25 %wt or less, or 20 %wt or less, exhibited relatively high elongation at break under dry conditions of 50% or higher, 100 % or higher, and even 300 % or higher or 500 % or higher.

In some embodiments, the elastomeric matrix provided herein is characterized under dry conditions by a tensile strength in the range 0.01 MPa to 1, 2, 3, 4, or 10 MPa. The module of elasticity (Young module) varies within similar ranges.

In the table below (Table 2), summarized are some mechanical properties measured under dry conditions with matrices that all had the same composition, except for differing in the types of PVOH.

TABLE 2

| PVOH | #1 | #2 | #3 | #4 | #5 |
|---|---|---|---|---|---|
| LCFH/total | 1 | 0.5 | 0.4 | 0.5 | 0.4 |
| SCPH1/total | 0 | 0.5 | 0.6 | 0 | 0 |
| SCPH2/total | 0 | 0 | 0 | 0.5 | 0.6 |
| Module Young (tension)[MPa] | 0.5±0.05 | 0.17±0.02 | 0.07±0.001 | 0.17±0.06 | 0.030±0.005 |
| Tensile strength [MPa] | 1.04±0.01 | 0.19±0.04 | 0.07±0.02 | 0.22 ±0.04 | 0.050±0.002 |
| Elongation at break [%] | 467±1 | 165±61 | 123±57 | 212±25 | 188±28 |
| Compressive module [MPa] | 0.63 | 0.48 | 0.29 | 0.51 | 0.22 |

These examples exemplify that increasing the amount of short chain partially hydrolyzed PVOH may lower elastic module, tensile strength, and elongation at break. In these examples, the difference between PVOH of the same hydrolysis degree (88%) and slightly different chain length (750 and 630) was reflected in modest changes in mechanical properties.

Mechanical measurements presented herein were performed using computer-controlled Mark10 model F105 tension/compression test frame, equipped with 10N or 100N force sensor equipped with standard grips and head. Software: "IntelliMESUR".

Wet measurements were performed in Simulated tear fluid (STF) with pH adjusted to ~7.2.

Tensile measurements done using "Dog bone" model. Dog bone dimensions: total length 6 cm. Grip width 2 cm, neck width 0.8 cm, thickness 0.5-3.5 mm. Measurement speed 40mm/min.

Tensile measurements of wet samples were performed after soaking a "dog bone" sample in 5 ml STF. After designated time the dog bone was taken out of the liquid, mounted on the grips and measured immediately.

Press measurements performed using 12.7mm flat head and cylinder shaped samples. Sample dimensions: 4mm diameter, height 2.5-3.0 mm. Measurement speed 20 mm/min.

A set of individual samples were soaked separately in small containers and each was measured after designated time. The samples were pressed inside the liquid without being touched or moved until the measurement.

An exemplary elastomeric matrix, the dry material of which was composed of 20% PVOH, all of which 28-99, 20% polyethylene glycol, and 60% glycerol, was measured to have Young Modulus of 0.41 MPa, tensile strength of 0.51 MPa, and elongation at break of 456 %.

Exemplary Plasticizers

The term "plasticizer", as used herein, refers to a wide range of substances that together with PVOH bestow the mechanical properties of the elastomeric matrix provided herein. The term "plasticizer", as used herein, refers to a wide range of substances that together with PVOH bestow the mechanical properties of the elastomeric matrix provided herein. Without being bound by any particular theory, it is assumed that the PVOH and at least one of the plasticizer(s) interact and form a hydrogen-bonded network that allows the matrix to exhibit shape stability and elasticity under wet and dry conditions. The hydrogen bonded network may include plasticizer molecules linked to two (or more) PVOH residues.

In some embodiments of the present invention, the plasticizer is an organic material, i.e., matter in its various forms that contain carbon atoms.

According to embodiments of the present invention, at least one of the plasticizers is characterized by exhibiting at least two hydrogen bond forming functional groups, namely at least two H-bond acceptors, at least two H-bond donors, or at least one H-bond acceptor and at least one H-bond donor.

According to some embodiments of the present invention, a plasticizer exhibits more than two H-bond forming functional groups, or more than 3, more than 4, more than 5, more than 6, more than 7, or more than 8 H-bond forming functional groups. In some embodiments, a plasticizer comprises a plurality of H-bond forming functional groups.

According to some embodiments, a plasticizer is characterized by a molar mass of less than 1,000 g/mol, or less than 500 g/mol. In some embodiments wherein the elastomeric matrix includes at least two plasticizers, the matrix may include at least one plasticizer which is an oligomer characterized by a molar mass of more than 1,000 g/mol. In some embodiments, the oligomer is characterized by a molar mass that ranges 1,000-2000 g/mol.

According to some embodiments of the present invention, a plasticizer is characterized by forming non-rigid crosslinked bridges with other elements of the matrix, such as PVOH. In this context, a plasticizer is characterized by exhibiting at least one, or at least two rotatable bonds in its structure, not including the bonds connecting the hydroxyl group to the molecule. In some embodiments, a plasticizer is characterized by exhibiting at least one bond having a variable dihedral angle. A dihedral angle is the angle between half-planes through two sets of three atoms, having two atoms in common. In some embodiments, the dihedral angle is defined between half-planes through two sets of three non-hydrogen atoms, having two non-hydrogen atoms in common. For example, ethylene glycol exhibits one variable dihedral angle which is defined by two sets of three atoms, each having an oxygen and two carbon atoms common to the two sets of non-hydrogen atoms.

In some embodiments, the plasticizers are biocompatible, namely the plasticizers do not elicit a non-tolerable local or systemic effect in a recipient/user. In some embodiments, a plasticizer is an ophthalmic compatible (acceptable) substance, namely it is biocompatible, and particularly does not elicit a non-tolerable ophthalmic effect in the recipient/user.

In some embodiments, the plasticizer(s) interact and form a hydrogen-bonded network that allows the matrix to exhibit shape stability and elasticity under wet and dry conditions. The hydrogen bonded network may include plasticizer molecules linked to two (or more) PVOH residues.

In some embodiments of the present invention, the plasticizer is an organic material, i.e., matter in its various forms that contain carbon atoms.

According to embodiments of the present invention, at least one of the plasticizers is characterized by exhibiting at least two hydrogen bond forming functional groups, namely at least two H-bond acceptors, at least two H-bond donors, or at least one H-bond acceptor and at least one H-bond donor.

According to some embodiments of the present invention, a plasticizer exhibits more than two H-bond forming functional groups, or more than 3, more than 4, more than 5, more than 6, more than 7, or more than 8 H-bond forming functional groups. In some embodiments, a plasticizer comprises a plurality of H-bond forming functional groups.

According to some embodiments, a plasticizer is characterized by a molar mass of less than 1,000 g/mol, or less than 500 g/mol. In some embodiments wherein the elastomeric matrix includes at least two plasticizers, the matrix may include at least one plasticizer which is an oligomer characterized by a molar mass of more than 1,000 g/mol. In some embodiments, the oligomer is characterized by a molar mass that ranges 1,000-2000 g/mol.

According to some embodiments of the present invention, a plasticizer is characterized by forming non-rigid crosslinked bridges with other elements of the matrix, such as PVOH. In this context, a plasticizer is characterized by exhibiting at least one, or at least two rotatable bonds in its structure, not including the bonds connecting the hydroxyl group to the molecule. In some embodiments, a plasticizer is characterized by exhibiting at least one bond having a variable dihedral angle. A dihedral angle is the angle between half-planes through two sets of three atoms, having two atoms in common. In some embodiments, the dihedral angle is defined between half-planes through two sets of three non-hydrogen atoms, having two non-hydrogen atoms in common. For example, ethylene glycol exhibits one variable dihedral angle which is defined by two sets of three atoms, each having an oxygen and two carbon atoms common to the two sets of non-hydrogen atoms.

In some embodiments, the plasticizers are biocompatible, namely the plasticizers do not elicit a non-tolerable local or systemic effect in a recipient/user. In some embodiments, a plasticizer is an ophthalmic compatible (acceptable) substance, namely it is biocompatible, and particularly does not elicit a non-tolerable ophthalmic effect in the recipient/user.

In the context of the present invention, according to some embodiments the elastomeric matrix comprises one or more plasticizers, and in other embodiments, the elastomeric matrix comprises at least two plasticizers. It is noted that when referring herein to a particular characteristic of a plasticizer, it is meant to be taken as referring to a single plasticizer or to each of the plurality of plasticizers individually.

According to some embodiments of the present invention, when the matrix includes more than one plasticizer, one is characterized by a viscosity that is at least 5-times higher than the viscosity of the other plasticizer. In some embodiments, at least one of the plasticizers is characterized by a viscosity of at least 1000 cp, and the other plasticizer is characterized by a viscosity of less than 200 cp. Alternatively, one plasticizer has a viscosity of 500 cp or above and the other 50 cp or below. For example, in one exemplary matrix, one of the plasticizers is glycerol, exhibiting a viscosity of about 1400 cp, and the other plasticizer is polyethylene glycol, exhibiting a viscosity of about 100 cp. In another example, one of the plasticizers is glycerol, and the other propylene glycol, exhibiting viscosity of about 40 cp. It is noted that when referring herein to a particular characteristic of a plasticizer, it is meant to be taken as referring to a single plasticizer, some of the plasticizers, or to each of the plurality of plasticizers individually.

According to some embodiments of the present invention, when the matrix includes more than one plasticizer, one is characterized by a viscosity that is at least 5-times higher than the viscosity of the other plasticizer. In some embodiments, at least one of the plasticizers is characterized by a viscosity of at least 1000 cp, and the other plasticizer is characterized by a viscosity of less than 200 cp. Alternatively, one plasticizer has a viscosity of 500 cp or above and the other 50 cp or below. For example, in one exemplary matrix, one of the plasticizers is glycerol, exhibiting a viscosity of about 1400 cp, and the other plasticizer is polyethylene glycol, exhibiting a viscosity of about 100 cp. In another example, one of the plasticizers is glycerol, and the other propylene glycol, exhibiting viscosity of about 40 cp. All viscosities are of the liquid plasticizers, at 20°C.

According to some embodiments, all plasticizers are liquid at room temperature.

In some embodiments, the one or more plasticizers constitutes at least 30 %wt, 40 %wt, 50 %wt, 60 %wt, 70 %wt, 80 %wt of the total weight of the matrix. In some embodiments, the plasticizer constitutes 30-80 %wt of the weight of the matrix and any sub-range therbetween.

In some embodiments, the plasticizer is selected from polyols (e.g. ethylene glycol, diethylene glycol (DEG), triethylene glycol (TEG), and tetraethylene glycol), propylene glycols, glycerol, esters of glycerol (e.g. triacetin), polyprotic organic acids, e.g., oxalic acid, maleic acid, citric acid, etc.), polyamines (e.g., spermine, spermidine, diethylenetriamine, triethylenetetramine, tris(2-aminoethyl)amine, a polyethylenimine (PEI; polyaziridine, etc.), Trypan blue, alkyl gluceths, aliphatic polyether glycos (e.g., polyethylene glycol, prolypropylene glycol, polysorbate 80), polyoxyethylenes, ethanolamines, erythritols, tromethamine, urea, saccharides, amino acids (e.g., glycine, aspartate/aspartic acid etc.), polyphenols (e.g. tannic acid) and any combinations thereof.

In some embodiments, the plasticizer is an ophthalmic lubricant, emollient or demulcent, as described by the FDA in 21 CFR 349.12. In the present document, the terms lubricant and demulcent are used interchangeably.

Examples of lubricants that may be used as plasticizers include dextran, gelatin, povidone, hyaluronic acid or pharmaceutically acceptable salt thereof, polyols, cellulose and cellulose derivatives.

Examples of polyols include glycerol, polyethylene glycol (e.g., PEG 300 or PEG 400), propylene glycol, and polysorbate (e.g., polysorbate 70).

Examples of cellulose derivatives include carboxymethyl cellulose sodium, hydroxyethyl cellulose, methylcellulose, and hydroxypropyl methylcellulose (HPMC).

Thus, according to some embodiments of the present invention, the plasticizers are ophthalmic demulcents selected from the group consisting of cellulose derivatives, carboxymethylcellulose sodium, hydroxyethyl cellulose, hypromellose, hydroxypropyl cellulose, methylcellulose, hemicellulose, dextran, gelatin, liquid polyols, glycerin, polyethylene glycol 300, polyethylene glycol 400, polysorbate 80, propylene glycol, povidone, and any combination thereof. While in some embodiments, an ophthalmic demulcent may be considered a pharmaceutically active ingredient, whenever a drug product or ophthalmic device is described in the present disclosure and claims as comprising both an API and ophthalmic demulcents, an ophthalmic demulcent, for example, each of the following ophthalmic demulcents, is not considered an API: PVOH, dextran, gelatin, povidone, hyaluronic acid or pharmaceutically acceptable salt thereof, glycerol, polyethylene glycol (e.g., PEG 300 or PEG 400), propylene glycol, polysorbate (e.g., polysorbate 70), carboxymethyl cellulose sodium, hydroxyethyl cellulose, methylcellulose, and hydroxypropyl methylcellulose (HPMC).

In some embodiments, the matrix comprises glycerol as a single plasticizer. In some embodiments, the matrix comprises propylene glycol as a single plasticizer. In some embodiments, the matrix comprises glycerol and propylene glycol as plasticizers. In some embodiments, the matrix comprises glycerol and propylene glycol, each alone or a mixture thereof mixed with PEG, as plasticizers. In some embodiments, the matrix comprises glycerol and propylene glycol, each alone or a mixture thereof mixed with PEG, as plasticizers. In some of embodiments, PEG is the amount of PEG and the amount of PVOH are substantially the same.

Exemplary compositions of elastomeric matrices

The invention further provides a composition of an elastomeric matrix immersed in liquid and/or combined with additional one or more elastomeric matrix(es), each of the matrixes being as disclosed herein. In some embodiments, a medical device, for example, for eluting drug, may comprise such a combination of matrices. The medical device may be, in some embodiments, an ophthalmic device, a buccal device, or medical device for use with other tissues.

In some embodiments, a medical device comprises two matrices as described therein, which differ from one another in their composition. For example, each of the two matrices may include different types of PVOH. In some embodiments, the two matrices may differ in the mass ratio between PVOH and the matrix. In some embodiments, the two matrices may differ in the types of PVOH included in each, and in some embodiments, the two matrices may differ in the mass ratio between the two types of PVOH. In some embodiments, the two matrices differ in more than one of PVOH to matrix mass ratio, types of PVOH, and mass ratio between types of PVOH.

In some embodiments a composition of elastomeric matrices comprises two or more matrices, each having a different composition of PVOH. For example, two of the compositions may differ in the weight percent of PVOH in the matrix, in the different PVOH types included in the matrix, and/or in the weight percent of each PVOH type out of the total PVOH of the matrix. In some embodiments, a composition of elastomeric matrices may comprise two or more matrices, each having distinct mechanical characteristics under dry conditions. In some embodiments, each of the elastomeric matrices degrades at a different rate and/or have a different time development of one or more mechanical characteristics under wet conditions. In some embodiments, the two matrices touch across a touching surface, which may be open or closed.

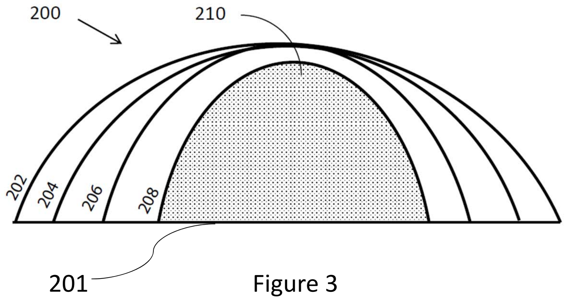

Referring to Figure 3, showing a simplified schematic cross sectional view of a medical device **200,** according to some embodiments of the invention. In some embodiments, medical device **200** is configured to residence on an eye surface under an eyelid. In some ophthalmic embodiments, device **200** has an anterior surface (e.g., **202**), that when worn on the eye is adjacent to the eyelid, and a posterior surface **201,** that when worn on the eye is adjacent to the eye surface (e.g., to the sclera).

In some embodiments, contours **202, 204, 206, 208,** illustrate the anterior surface of device **200** at different times, e.g. as device **200** degrades during residence on an eye, starting with contour **202** and finishing with contour **208.** In some embodiments, contour **208** defines a core **210** of device **200.**

In some embodiments, device **200** includes different portions that degrade at different rates. Where, in some embodiments, core **210** includes material which is less rapidly degraded (or is not degraded within the eye) than other portions of the device. In some embodiments, different portions of device **202** comprise matrices of different compositions. In one example, a less rapidly degraded portion of the device (e.g. core portion **210**) is made of a matrix having more PVOH than the other portions, or, in another example, the less rapidly degraded portion has the same amount of PVOH as other portions, but more of that PVOH is fully hydrolyzed. In some embodiments, core **210** differs from other parts of device **200** in other aspects of the composition, for example, different amounts of PVOH and different composition of PVOH types. In some embodiments, core **210** touches other portions of the device across an open, dome-like touching surface **211.**

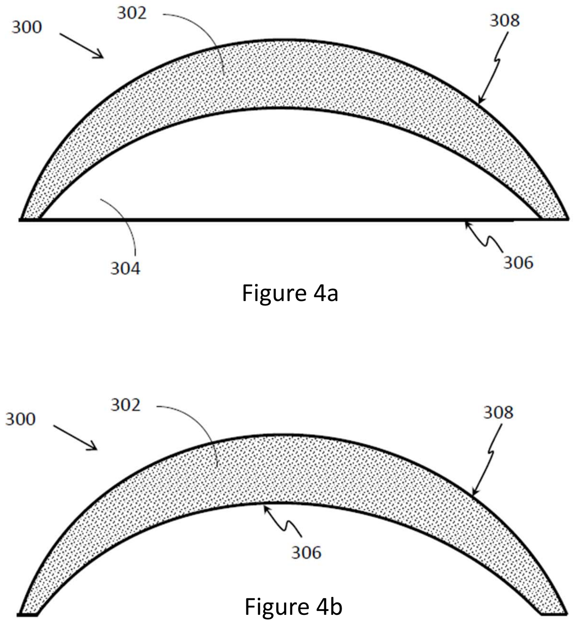

Referring now to Figures 4a-b, showing simplified schematic cross sectional views of medical device **300,** according to some embodiments of the invention. In some embodiments, medical device **300** may be an ophthalmic device. In some embodiments, medical device **300** may be a buccal device. In the following, device **300** is referred to as an ophthalmic device configured to reside between an eye surface and an eyelid.

In some embodiments, device **300** includes different parts **302, 304** with different degradation times. Parts **302** and **304** are shown touching across surface **311.** While device **200** illustrates a device in which both the anterior and the posterior surfaces change contour due to degradation under wet conditions, in device **300,** only posterior surface **306** changes contour. In some embodiments, this is because the two parts of device **300,** namely, posterior part **304** and anterior part **302,** are made of different matrices, each having respective degradation properties. Thus, in some embodiments, anterior part **302** barely changes while posterior part **304** fully degrades, so that posterior surface **306** changes contour from the one depicted in Figure 4a (in which posterior surface is shown as flat) to the one depicted in Figure 4b (in which the posterior surface is shown to unite with touching surface **311**).

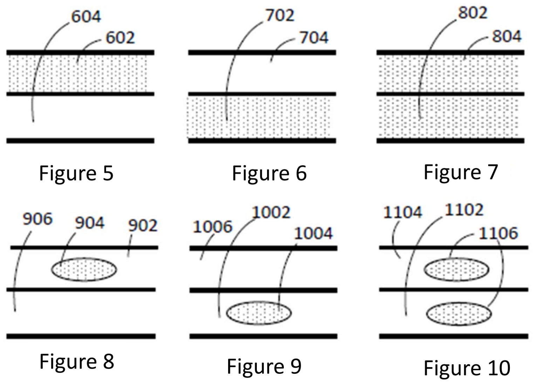

Referring now to Figures 5, 6, 7, 8, 9 and 10 showing simplified schematic cross sectional views of portions of ophthalmic devices, according to some embodiments of the invention. The ophthalmic devices schematically illustrated in Figures 5 to 10 provide examples to the richness in drug release profiles that may be achieved with medical devices comprising two or more matrices as described herein, and more generally, to the richness in drug release profiles enabled by embodiments of the present invention.

In Figures 5-8, shaded areas indicate presence of therapeutic ingredient/s, according to some embodiments.

Referring now to Figure 5 and Figure 8, in some embodiments, a second layer **602, 902** comprises therapeutic ingredient/s. In some embodiments, layer **602** and/or layer **902** is configured to contact, at least periodically, an inner surface of an eyelid, and/or forms at least part of anterior surface. In some embodiments, layer **602** and/or layer **902** has a smooth outer surface and/or includes lubricious material.

In Figure 5, the therapeutic agents are shown dispersed through layer **602** that touches layer **604** across open touching surface **611,** while in Figure 8, the therapeutic agent is illustrated to be dispersed only through one or more discrete regions **904** within layer **902**. Discrete regions **904** are also referred to herein as pellets, and touch layer **902** across a closed, ellipsoidal, touching surface **911**. Layers **604** and **906** are free of therapeutic ingredient/s. In some embodiments, discrete regions **904** are made of an elastomeric matrix of different degradation properties than those of layer **902** hosting it. Thus, layer **902** may first degrade, to release pellets **904,** which degrade gradually after being released. This arrangement may cause a delayed release of the therapeutic agent, which is not released until layer **902** is degraded, and then released slowly, as pellets **904** degrade.

Referring now to Figure 6 and Figure 9, they show embodiments similar to those of Figure 5 and Figure 8, respectively, but with the therapeutic agent being dispersed in posterior parts of the depicted ophthalmic devices, rather than in the anterior parts.

Referring now to Figure 7 and Figure 10, in some embodiments depicted therein, the therapeutic agent is dispersed throughout both anterior parts **804** and **1104** and posterior parts **802** and **1102.** In some embodiments, each part in Figure 7 is made of a different matrix, thus releases the therapeutic agent at a different pace. In Figure 10, pellets **1106** may be made of a matrix different from the matrix around them, and optionally, different from the matrix making the other part.

In some embodiments, a composition of the invention comprises at least two elastomeric matrices as disclosed herein, which may be similar or different in composition, constitution and properties. In some embodiments, one formulation may be cast into a mold and let dry to provide an elastomer, and another solution may be cast above said elastomer to provide a bilayer elastomeric composition of matrices. In some embodiments, a plurality of minute elastomers of one kind (or of several kinds) may be mixed with a formulation that when settles provides an elastomer of another kind, so when the formulation settles it provides a "raisin cake" composition of the one or more elastomeric minute samples in a "dough" of another elastomer (as illustrated, for example, in Figures 8, 9, and 10.

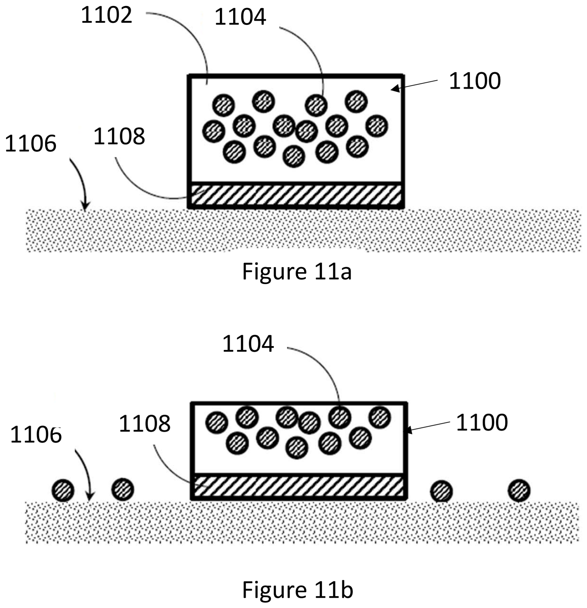

Referring now to Figures 11a-11b, showing simplified schematic cross sectional views of a device on an eye surface, according to some embodiments of the invention.

For simplicity, device **1100** is illustrated as having a rectangular cross section, however device **1100,** in some embodiments, has a shape (shape e.g. including curvature of a posterior and/or anterior surface of the device) having one or more feature of device/s described elsewhere within this document.

In some embodiments, device **1100** includes a layer **1102** which includes therapeutic material particles **1104.** Where material particles **1104,** in some embodiments, include one or more feature of microparticles and/or nanoparticles.

In some embodiments, material particles **1104** are devices having retention portions. Where, in some embodiments, device **1100** includes 1-10 particles **1104** e.g. each having a retention portion.

Where, in some embodiments, other material of layer **1102** is rapidly dissolvable and/or degradable and/or erodible. Erosion, in some embodiments, revealing and allowing dispersion of particles **1104,** for example, as shown in transition between Figure 11a to Figure 11b. In some embodiments, e.g. alternatively to what is illustrated in Figure 11b, particles are dispersed within the eyelid and remain underneath the eyelid, for example, retention portions meaning that eye tissue retains the particles **1104** underneath the eyelid.

In some embodiments, particles **1104** include therapeutic material. Optionally, in some embodiments particles **1104** include mucoadhesive material. Potentially, once a particle is released from layer **1102** if it contacts eye surface **1106** (e.g. under movement/s of one or more of the eyelid, eyeball, and tear fluid) it adheres to the eye surface. In some embodiments, particles adhere to one or more mucosal surface e.g. including the eyeball and/or eyelid. In some embodiments, the particles then degrade (e.g. over about 10 minutes or about 1 hour, or about 12 hours, or about 1 day, or about 3 days, or lower or higher or intermediate time durations) to release therapeutic material to eye tissue.

Optionally, in some embodiments, device includes one or more additional layer (e.g. than layer **1102**). For example, in some embodiments, device **1100** includes a mucoadhesive layer **1108.**

In some embodiments, therapeutic material layer **1102** including particles is incorporated into one or more of the device embodiments as described within this document. Including, for example, covering and/or access features to therapeutic material layer **1102.**

In some embodiments, the composition of the invention comprises a solution comprising at least one plasticizer, with at least one elastomeric matrix being immersed in said solution. In some embodiments, said composition of the invention further comprises a fluid selected from water or water-based solution or solvent, wherein said at least one elastomeric matrix is immersed in said fluid.

In some embodiments, said composition is stored, prior to use thereof, under dry conditions.

While the elastomeric matrix provided herein can be prepared to suit the desired properties, some applications require the matrix to exhibit unique properties, which require to add another type of polymer(s) which is not PVOH of any type, yet typically produced from hydrogen-forming monomers. This type of elastomeric matrices is referred to herein as a composite matrix.

In general, composite materials are the products of a connection between two different chemical entities. The connection between the different entities can be through covalent, non-covalent, ionic or other type or bond. The resulting properties sometimes represent a simple or complex weighted average of the individual properties of each ingredient, and sometimes products with very different properties from those of the two original components are obtained.

In the context of the present invention, it was found that by adding one or more polymers that are not PVOH of any type, new materials with a myriad of diverse characteristics can be obtained while keeping the basic characteristics of the elastomeric matrix provided herein, namely low amount of polymers, softness, and shape retention under varying dry and wet conditions.

The addition of the non-PVOH polymer can be used for example in order to further modify one or more of stability in dry and/or wet conditions, bio-degradability, rheological properties, bio-adhesiveness, compatibility with active materials, active material release profile and imprinting active materials.

In some embodiments, the composite elastomeric matrix is made by blending PVOH of one or more types with a non-PVOH polymer capable of hydrogen bonding with PVOH. In some embodiments, said non-PVOH polymer is neutral (not charged). In some embodiments, said non-PVOH polymer has anionic or cationic functional groups (charged). In some embodiments, said non-PVOH polymer is a synthetic polymer. In some embodiments, said non-PVOH polymer is an oligosaccharide or polysaccharide. In some embodiments said non-PVOH polymer is an acrylic polymer.

It is clear to those skilled in the art that additional polymers and various types of molecular links (e.g. ionic, complexes etc.) can be used to create composite materials with PVOH of one or more types, using either covalent or non-covalent interactions in order to connect the two polymers. The additional polymer that is not PVOH can be selected from various polymers, including naturally occurring polymers and macromolecules, synthetic polymers and the likes. For example, without limitation, the polymer of hydrogen bond forming monomers that is not PVOH is a polyacrylic acid, a polyvinyl pyrrolidone, cellulose, chitin, glycogen, starch, gellan, dextran, inulin, pectin, arabinoxylan, and any mixture thereof.

According to some embodiments of the present invention, the mass content of the non-PVOH polymer in the elastomeric matrix provided herein is smaller than the mass content of PVOH of all the types together. In some embodiments, the mass ratio between PVOH (of the one or more types) and the non-PVOH polymer ranges from about 100: 1 to 2:1. Some exemplary embodiments of composite matrices are provided in the Examples section that follows below.

Exemplary Medical/Ophthalmic device

The present invention further includes the aspect of a medical device (e.g., an ophthalmic/ocular device) that can be used in treating pathologies and diseases, wherein the device comprises or consists of the elastomeric matrix provided herein. In some embodiments, the device may be used topically, in a body cavity, or the like. In some embodiments, the device may be injected into a body part or tissue.

In the context of the ophthalmic device, according to some embodiments of the present invention, the matrix comprises or consists of ophthalmically acceptable ingredients. For example, any ingredient allowed or approved for ophthalmic use by any regulatory authority may be referred to as ophthalmically acceptable ingredients. In some embodiments, all the plasticizers are ophthalmic demulcents. While in some embodiments, an ophthalmic demulcent may be considered a pharmaceutically active ingredient, whenever a drug product or ophthalmic device is described in the present disclosure and claims as comprising both an API and ophthalmic demulcents, an ophthalmic demulcent, for example, each of the following ophthalmic demulcents, is not considered an API: PVOH, dextran, gelatin, povidone, hyaluronic acid or pharmaceutically acceptable salt thereof, glycerol, polyethylene glycol (e.g., PEG 300 or PEG 400), propylene glycol, polysorbate (e.g., polysorbate 70), carboxymethyl cellulose sodium, hydroxyethyl cellulose, methylcellulose, and hydroxypropyl methylcellulose (HPMC).

In some embodiments, the elastomeric matrix provided herein is particularly useful for sequestering and/or releasing active pharmaceutical ingredients (API), since it is unreactive, stable and benign. In some embodiments, the ophthalmic device that comprises the elastomeric matrix provided herein, is configured for drug delivery, both for bolus as well as for sustained release regimens. Hence, according to some embodiments, the matrix includes therein or thereon at least one active pharmaceutical ingredient (API). Optionally, the active pharmaceutical ingredient (API) is selected for treating an ophthalmic symptom, disease or disorder. Optionally, the active pharmaceutical ingredient (API) is selected for treating a buccal symptom, disease or disorder (see below).

In some embodiments, the ophthalmic device described herein is generally sized and shaped to be positioned on an outer surface of the eye with at least a portion of the ocular device positioned under one eyelid in a manner that does not contact or interfere with the cornea. In some embodiments, the ophthalmic device provided herein is configured to be positioned on a surface of the eye, at least partially underneath the upper or lower eyelid and outside a cornea of the eye for delivering at least one active pharmaceutical ingredient (API) to an eye for an extended period of time, for example of between 5 minutes and 24 hours. Optionally more than 24 hours.

In some embodiments, the elastomeric matrix provided herein is characterized by a porous microstructure, which allows entrapping a liquid therein. In some embodiments, the liquid may be, for example, a solution or a colloid comprising an active pharmaceutical ingredient (API), and this liquid can be dispersed in the matrix and/or entrapped in the pores of the matrix. Hence, in some embodiments, the device includes at least one active pharmaceutical ingredient (API) dispersed, sequestered, impregnated within the elastomeric matrix provided herein.

In some embodiments, the active pharmaceutical ingredient (API) can include, for example, a small molecule, a macromolecule, a cell or a tissue.

In some embodiments, the active pharmaceutical ingredient (API) is in a liquid form. In some embodiments, the active pharmaceutical ingredient (API) is in solid form. In some embodiments, the active pharmaceutical ingredient (API) is soluble in water, or soluble in an organic solvent, or amphiphilic.

In some embodiments, the active pharmaceutical ingredient (API)l s encapsulated, or microencapsulated, or in a microparticle form, or in a nanoparticle form.

In the context of the present invention, the active pharmaceutical ingredient (API) is, without limitation, an analgesic agent, an antacid, an antianxiety agent, an antiarrhythmic, an antibacterial agent, an antibiotic agent, an anticoagulant, a thrombolytic agent, an anticonvulsant, an antidepressant, an antiemetic agent, an antifungal agent, an antihistamine, an antihypertensive, an anti-inflammatory agent, an antineoplastic agent, an antpsychotic agent, an antipyretic agent, an antiviral agent, a barbiturate, a bronchodilator, a beta-blocker, a corticosteroid, a cold cure, a cytotoxic agent, a decongestant, a diuretic agent, a expectorant, a hormone, a hypoglycemic agent, an immunosuppressive agent, a laxative, a muscle relaxant, a sedative, a sex hormone, a sleeping drug, a tranquilizer, a vitamin, and any combinations thereof.

In some embodiments, the active pharmaceutical ingredient (API) is an ophthalmological active pharmaceutical ingredient (API). In some embodiments, the ophthalmological active pharmaceutical ingredient (API) is a lubricant, an anti-angiogenic agent, a mydriatic agent, an anesthetic, an anti-infective agent, an anti-inflammatory agent, an antihistamine, a glaucoma treatment agent, a surgical agent, a diagnostic agent or any combination thereof.

While in some embodiments, an ophthalmic demulcent may be considered a pharmaceutically active ingredient, whenever a drug product or ophthalmic device is described in the present disclosure and claims as comprising both an API and ophthalmic demulcents, an ophthalmic demulcent, for example, each of the following ophthalmic demulcents, is not considered an API: PVOH, dextran, gelatin, povidone, hyaluronic acid or pharmaceutically acceptable salt thereof, glycerol, polyethylene glycol (e.g., PEG 300 or PEG 400), propylene glycol, polysorbate (e.g., polysorbate 70), carboxymethyl cellulose sodium, hydroxyethyl cellulose, methylcellulose, and hydroxypropyl methylcellulose (HPMC).

In some embodiments, the active pharmaceutical ingredient (API) can include, without limitation, bimatoprost, travoprost, latanoprost, tafluprost, NSAID, steroid, antihistamine, carbonic anhydrase inhibitor (CAI), dorzolamide, cyclosporine, antibiotic, doxycycline, tetracycline, azithromycin, fatty acid, long chain fatty acid, fatty alcohol, cetyl alcohol, stearyl alcohol, non-penetrating steroid, free acid of steroid, lipid, ketorolac, silicone oil, olopatadine, prostaglandin, prostaglandin analog, prostamide, small-molecule integrin antagonist, lifitegrast, loteprednol, and fluoromethalone or a combination thereof.

In some embodiments, the active pharmaceutical ingredient (API) can include a prostaglandin analogue. In some embodiments, the prostaglandin analogue can include at least one of bimatoprost, latanoprost, travoprost, and tafluprost. In some embodiments, the active pharmaceutical ingredient (API) can be for lowering the intraocular pressure of the eye. In some embodiments, the active pharmaceutical ingredient (API) can be for treating dry eye. In some embodiments, the active pharmaceutical ingredient (API) can include at least one of cyclosporine, steroid, loteprednol, fluoromethalone, non-penetrating steroid, free acid of steroid, nonsteroidal anti-inflammatory, ketorolac, small-molecule integrin antagonist, lifitegrast, doxycycline, azithromycin, lipid, fatty alcohol, cetyl alcohol, stearyl alcohol, fatty acid, long chain fatty acid, oil, or silicone oil. In some embodiments, the active pharmaceutical ingredient (API) can include a steroid. The steroid can include at least one of loteprednol or fluoromethalone.

In some embodiments, when preparing an ophthalmic device that comprises the elastomeric matrix according to some embodiments of the present invention, a formula is prepared according to the preparation procedure provided below. Briefly, a liquid formula is obtained which can be used to prepare the final device. The active pharmaceutical ingredient (API) can be mixed into said liquid formula which leads to a matrix and a device that includes the active pharmaceutical ingredient (API). Hydrophilic active pharmaceutical ingredients (API) easily dissolve in the formula, whereas hydrophobic active pharmaceutical ingredients (API) may be suspended (e.g., as a colloid) in the formula.

The amount of the active pharmaceutical ingredient (API) in the matrix depends on the active pharmaceutical ingredient (API) and the treatment regimen. The nature of the matrix and the requirement therefrom, allow the matrix to include up to 30 % of its non-water constituents to be active pharmaceutical ingredients (API). For example from about 5 % to about 25 %, optionally form about 3 % to about 27 %, or any percentage between 0 % and 30 %.

In some embodiments, the matrix of the device further comprises at least one ophthalmically acceptable carrier/supplement/adjuvant/additive/non-pharmaceutical agent.

In some embodiments, the drug release profile depends on the active pharmaceutical ingredient (API), and the regimen. A skilled artisan would appreciate the means and technology present available for controlling sustained release of drugs from various devices and under various conditions. For example, encapsulation of active pharmaceutical ingredients (API), addition of materials that modifies the interaction between the agent and the matrix, and the likes.

In some embodiments, the active pharmaceutical ingredient (API) is released from the matrix when the matrix is in the presence of a liquid, such as water, saline, tear simulating fluid, saliva, blood, or any body fluid. When referring to "presence of liquid" it should be understood to relate to the condition wherein the matrix of the invention is exposed to liquid, partially or entirely immersed therein.

When referring to "presence of liquid" it should be understood to relate to the condition wherein the matrix of the invention is exposed to liquid, partially or entirely immersed therein.

In some embodiments, the active pharmaceutical ingredient (API) is maintained within the elastomeric matrix under dry conditions. In some embodiments, the active pharmaceutical ingredient (API) is maintained within the matrix during storage. In some embodiments, the active pharmaceutical ingredient (API) is maintained within the matrix under dry conditions for at least six months.

Exemplary method of treatment

In some embodiments, the elastomeric matrix of the present invention can be used for administration of active pharmaceutical ingredients (API) to a cell, tissue, or membrane. The invention further provides a method of administering at least one active pharmaceutical ingredients (API) to a cell or a tissue/membrane, which is realized by contacting the cell or tissue/membrane with a therapeutically effective amount of the active pharmaceutical ingredient (API) present in the elastomeric matrix as disclosed herein. In some embodiments, the tissue is selected from ocular, buccal, dental, orthodontic, muscular, mucosal, dermatological, connective, cardiac and any combinations thereof.

As used herein, the phrase "therapeutically effective amount" describes an amount of an active pharmaceutical ingredient (API) being administered, which will relieve to some extent one or more of the symptoms of the medical condition being treated. Thus, any amount may be therapeutically effective, depending on the condition to be treated, the effectiveness of the active pharmaceutical ingredient (API), the body size of the patient, etc. In the context of the present embodiments, the phrase "therapeutically effective amount" describes an amount of the active pharmaceutical ingredient (API) being administered and/or re-administered, which will relieve to some extent one or more of the symptoms of the condition being treated. In some embodiments, relieve may be achieved by being at a level that is harmful to the target cell(s) or microorganism(s), and cause a disruption to the life-cycle of the target cell(s) or microorganism(s). In some embodiments, relieve may be achieved by being at a level that is pharmaceutically effective to induce a therapeutic effect on a patient, for example, an effective quantity of muscle relaxant, pain reliever and/or antibiotics.

In the context of embodiments of the present invention, the therapeutically effective amount may refer to the active pharmaceutical ingredient (API) as a whole or to the amount of one or more active pharmaceutical ingredient (API) releasably sequestered and/or included in the matrix. The efficacy of any active pharmaceutical ingredient (API), can be determined by several methodologies known in the art.

According to another aspect of embodiments of the present invention, any one of the matrices described herein is suitable for use in treating a subject diagnosed with a medical condition treatable by at least one active pharmaceutical ingredient (API) sequestered (or included in the matrix) and controllably releasable from the matrix.

According to another aspect of embodiments of the present invention, there is provided a use of any of the elastomeric matrixes described herein as delivery vehicle for use as a medicament. In some embodiments, the medicament is for treating a subject diagnosed with a medical condition treatable by at least one of the drugs sequestered (or included in the matrix) and controllably releasable from the matrix.

In any of the methods and uses described herein, the matrix can be used for medicinal purposes as a part of a medical device.

Exemplary method of preparation

The present invention further provides a method of preparing the elastomeric matrix as disclosed herein, wherein the method comprises the steps of:

Option 1: Preparing individual solutions, each comprising a different type of PVOH. Mixing parts of the individual solutions according to the required ratio.
Option 2: Dissolving two or more types of PVOH in water to form a solution.

- Optionally heating the solution (either option 1 or option 2)
- Mixing the plasticizer(s) into the solution to form a mixture;
- Optionally heating the mixture;
- Adding the mixture into a mold;

- Allowing the mixture set in the mold, to thereby form the elastomeric matrix of the invention.

In some embodiments, preparing the PVOH solution may comprise preparing individual solutions, each comprising a different type of PVOH, and mixing parts of the individual solutions in amounts selected to achieve the required ratio between the different types of PVOH. Preparing each individual solution may include stirring the PVOH with water, while heating.

In some embodiments, the PVOH solution may be prepared by dissolving two or more types of PVOH in water, each at a respective weight, so that the ratio between the two types of PVOH in the solution will be as required. The dissolving preferably includes heating and mixing.

In some embodiments, the mixture has less than 20 %wt PVOH.

In some embodiments, the plasticizers make 30 %wt or more of the mixture.

In some embodiments of the method of preparing the matrix of the invention, the optional heating, as well as the heating applied in order to prepare the PVOH solution is to a temperature in the range of 60-100 °C.

In further embodiments of the method, adding the mixture into a mold is conducted at room temperature, for example, after the mixture is let to cool while mixing. In some embodiments of the method, adding the mixture into a mold is conducted in an open form, e.g., in an open flask or mold. In some embodiments, the elastomeric matrix is poured into the mold. In some embodiments, the elastomeric matrix is pumped into the mold.

In some embodiments of the method, at least one active pharmaceutical ingredient (API) is added to the solution, preferably, when the solution is at about room temperature, as not to encourage reactions between the active pharmaceutical ingredient (API) and other ingredients in the solution. In some embodiments, this also allows using active pharmaceutical ingredients (API) that are heat sensitive. In some embodiments, the API is added as is. In some embodiments, the API is dissolved or dispersed in a suitable liquid. What liquid is suitable may depend on the API. Examples of some suitable liquids include water and organic solvents, e.g., glycerol, and/or propylene glycol.

In some embodiments, the API is not heated at any stage of the method, and the elastomeric matrix or ophthalmic device containing it is obtained without heating the API.

In some embodiments of the method, the elastomeric matrix that is formed in step (f) is immersed, e.g., for 5 minutes, in a solution comprising at least one plasticizer or water or an aqueous solution or a solvent.

As used herein with reference to quantity or value, the term "about" means "within ± 10 % of".

The terms "comprises", "comprising", "includes", "including", "has", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the phrases "substantially devoid of" and/or "essentially devoid of" in the context of a certain substance, refer to a composition that is totally devoid of this substance or includes less than about 5, 1, 0.5 or 0.1 percent of the substance by total weight or volume of the composition. Alternatively, the phrases "substantially devoid of" and/or "essentially devoid of" in the context of a process, a method, a property or a characteristic, refer to a process, a composition, a structure or an article that is totally devoid of a certain process/method step, or a certain property or a certain characteristic, or a process/method wherein the certain process/method step is effected at less than about 5, 1, 0.5 or 0.1 percent compared to a given standard process/method, or property or a characteristic characterized by less than about 5, 1, 0.5 or 0.1 percent of the property or characteristic, compared to a given standard.

When applied to an original property, or a desired property, or an afforded property of an object or a composition, the term "substantially maintaining", as used herein, means that the property has not change by more than 20 %, 10 % or more than 5 % in the processed object or composition.

The term "exemplary" is used herein to mean "serving as an example, instance or illustration". Any embodiment described as "exemplary" is not necessarily to be construed as preferred or advantageous over other embodiments and/or to exclude the incorporation of features from other embodiments.

The words "optionally" or "alternatively" are used herein to mean "is provided in some embodiments and not provided in other embodiments". Any particular embodiment of the invention may include a plurality of "optional" features unless such features conflict.

As used herein, the singular forms "a", "an" and "the" include plural references unless the context clearly dictates otherwise. For example, the term "a compound" or "at least one compound" may include a plurality of compounds, including mixtures thereof.

Throughout this application, embodiments of this invention may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

As used herein the terms "process" and "method" refer to manners, means, techniques and procedures for accomplishing a given task including, but not limited to, those manners, means, techniques and procedures either known to, or readily developed from known manners, means, techniques and procedures by practitioners of the chemical, material, mechanical, computational and digital arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non limiting fashion.

Exemplary elastomeric matrix

EXAMPLE 1

### *Preparation of an elastomeric matrix*

The preparation of an elastomeric matrix according embodiments of the present invention, may start with the preparation of a PVOH solution.

Briefly, 420 ml of purified water were added into 500 ml round bottom flask equipped with an overhead stirrer. The flask was placed into a heating mantle, and 80 gr of PVOH were added in portions while stirring and heating until all the PVOH dissolved. Optionally, the PVOH included PVOH of two or more types, in the required mass ratio between them. See Table 7 for exemplary mass ratios between exemplary different types of PVOH.

The PVOH solution was left to stir and heat for additional 1-2 hours, and thereafter the solution was left to cool to room temperature while stirring.

Plasticizers and 80 gr of the PVOH solution were added in a 500 ml glass beaker to obtain a mixture according to table 3 (dry matter basis). Optionally purified water was added to dilute concentration of solutes. The mixture was heated with magnetic stirring until fully dissolved, and thereafter heating was turned off and the mixture was left to cool.

Table 3 presents some exemplary formulations for preparing exemplary elastomeric matrices as provided herein. The values represent mass content in percent of the total weight of non-water ingredients.

TABLE 3

| | | | | | | | | | | | | | | |
|---|---|---|---|---|---|---|---|---|---|---|---|---|---|---|
| **PVOH** | 33 | 25 | 20 | 20 | 20 | 15 | 15 | 15 | 15 | 10 | 10 | 10 | 10 | 5 |
| **Polyethylene glycol** | 35 | 25 | 25 | 20 | 15 | | 10 | 5 | 20 | 15 | 10 | 20 | 5 | 5 |
| **Glycerol** | 20 | 25 | 30 | 60 | 34 | 35 | 30 | 40 | 30 | 37 | 40 | 35 | 37.5 | 45 |
| **Propylene glycol** | 12 | 25 | 25 | | 30 | 35 | 30 | 40 | 31 | 34 | 40 | 35 | 37.5 | 45 |
| **Poly propylene glycol** | | | | | | 15 | | | | | | | | |

(continued)

| | | | | | | | | | | | | |
|---|---|---|---|---|---|---|---|---|---|---|---|---|
| **Kollicoat® IR** | | | | | | 15 | | | | | 10 | |
| **Tromethamine** | | | | 1 | | | | | | | | |
| **Dextran** | | | | | | | 4 | | | | | |

EXAMPLE 2

## Comparative study

In order to study the criticality of the mass ratio between PVOH and the plasticizers, 20 gr of PVOH were mixed with 36 gr glycerol in a 500 ml glass beaker, and water was added thereto in order to achieve a solution of 20 %wt PVOH. It is noted herein that this formulation for producing a matrix that is characterized by a mass content plasticizer to PVOH ratio of 36:20, namely 1:1.8, which is outside the scope of the present invention. The mixture was heated until complete dissolution. Heating was turned off and the solution was left to cool.

The resulting elastomeric matrix show inferior stability in both dry and wet conditions compared to a comparable matrix wherein the mass content ratio of plasticizer to PVOH is 2:1.

Another comparative example was prepared according to the procedure described hereinabove, using 35 %wt of high molecular weight PVOH, 11 %wt of polyethylene glycol, 27 %wt of glycerol, and 27 %wt of propylene glycol.

This formulation produced substances that exhibited swelling in water of more than 20 %, and have not retained the shape it had under dry conditions.

EXAMPLE 3

## Preparation of device

A solution of each of the various formulations specified in Table 5 was poured into a respective mold (open or closed) and left to dry. Optionally, a coating machine was used to create a film of desired thickness. The film was left to dry.

EXAMPLE 4

## Composite elastomeric matrices

To prepare a composite elastomeric matrix, according to some embodiments of the present invention, a formulation according to Table 3 was prepared and 10 gr of the formulation were mixed with a non-PVOH polymer in order to reach a ratio of polyvinyl alcohol to the non-PVOH polymer according to Table 4 below. The mixture was stirred until complete dissolution and further processed to create a device (e.g., a film).

TABLE 4

| Non-PVOH polymer | Ratio of PVOH to non-PVOH polymer |
|---|---|
| Sodium carboxymethyl cellulose (CMC Na) | 100:1 |
| Chitosan | 90:10 |
| Hydroxypropyl methylcellulose (HPMC) | 100:1 |
| Hydroxypropyl cellulose (HPC) | 100:1 |
| Polyvinyl pyrrolidone (PVP) | 75:25 |
| Polyacrylic acid | 75:25 |
| Dextran | 80:20 |

Exemplary elastomeric matrix comprising two or more types of PVOH

EXAMPLE 5

*Preparation of an elastomeric matrix*

The preparation of an elastomeric matrix according embodiments of the present invention, may start with the preparation of a PVOH solution comprising two types of PVOH. Optionally, the two (or more) types of PVOH together are mixed with the water as described below. Alternatively, two separate mixtures of PVOH are prepared, and then mixed together according to the required ratio between the two types of PVOH and the PVOH concentrations in the mixtures. Alternatively, one PVOH is prepared as a solution, and the other is added AS IS, for example with the plasticizers.

Briefly, 420 ml of purified water were added into 500 ml round bottom flask equipped with an overhead stirrer. The flask was placed into a heating mantle, and 80 gr of PVOH (e.g., 40 gr of PVOH of a first type and 40 gr of PVOH of a second type) were added in portions while stirring and heating until all the PVOH dissolved.

The PVOH solution was left to stir and heat for additional 1-2 hours, and thereafter the solution was left to cool to room temperature while stirring.

Plasticizers and 80 gr of the PVOH solution were added in a 500 ml glass beaker to obtain a mixture according to table 5 (dry matter basis). Optionally purified water was added to dilute concentration of solutes. The mixture was heated with magnetic stirring until fully dissolved, and thereafter heating was turned off and the mixture was left to cool.

Table 5 presents some exemplary formulations for preparing exemplary elastomeric matrices as provided herein. The values in rows 1, 7, 8, and 9 represent mass content in percent of the total weight of non-water ingredients. In table 1, the total amount of PVOH is provided in row 1. This amount may be divided between two or more types of PVOH, for example, as depicted in rows 2 to 6, showing the percentage of each type of PVOH out of the total amount of the PVOH, shown in row 1. In some embodiments, one type may be long chain highly hydrolyzed PVOH, and the other type may be short chain partially hydrolyzed. The ratio between them may be, for example, between 25% and 75% long chain fully hydrolyzed, and the balance short chain partially hydrolyzed. The inventors were surprised to see that while short chain partially hydrolyzed PVOH alone results in matrices of very low quality (e.g., when immersed in water, such matrices might swell non-isotropically, and add more than 100% to their volume), replacing as little as a quarter of the short chain partially hydrolyzed PVOH with long chain fully hydrolyzed PVOH was enough to provide satisfying matrices.

There are many types of PVOH commercially available, and in the following examples, PVOH from Merck's Emprove® product line was used, except for Sigma Aldrich 8.21039, which is PVOH for synthesis. Average molecular weight, chain length, and hydrolysis degree of the used products are provided in Table 1 above.

**LCFH1** and **LCFH2** are two different types of long chain fully hydrolyzed PVOH taken from the table above. Similarly, **SCPH1** and **SCPH2** are two different types of short chain partially hydrolyzed PVOH taken from the table above.

TABLE 5

| Table 5 | # | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|---|
| 1 | Total PVOH | 30 | 25 | 25 | 20 | 20 | 20 | 20 | 15 | 15 | 15 | 15 | 10 | 10 | 10 | 10 | 10 | 5 | 5 |
| 2 | %LCFH1 | 30 | 60 | 25 | 30 | 35 | 30 | 35 | 65 | 50 | 30 | 40 | 80 | 70 | 80 | 75 | 50 | 90 | 90 |
| 3 | %SCPH1 | | | | | | 70 | | | 50 | 70 | | 20 | | | | | 10 | |
| 4 | %SCPH2 | | | 75 | | | | 65 | | | | 60 | | 30 | 20 | 25 | | | |
| 5 | %LCFH2 | | 40 | | 70 | 65 | | | 35 | | | | | | | | 50 | | 10 |
| 6 | %LCPH | 70 | | | | | | | | | | | | | | | | | |
| 7 | Polyethylene glycol | 10 | 10 | 10 | 25 | 20 | 25 | 20 | 5 | 25 | 5 | 15 | 15 | 10 | 15 | 10 | 20 | 5 | 5 |
| 8 | Glycerol | 30 | 32.5 | 32.5 | 30 | 60 | 30 | 60 | 40 | 30 | 40 | 30 | 37 | 40 | 37 | 40 | 35 | 45 | 50 |
| 9 | Propylene glycol | 30 | 32.5 | 32.5 | 25 | | 25 | | 40 | 30 | 40 | 40 | 38 | 40 | 34 | 40 | 35 | 45 | 40 |

EXAMPLE 6

**Preparation of device**

A solution of each of the various formulations specified in Table 5 or in Table 7 was poured into a respective mold (open or closed) and left to dry. Optionally, a coating machine was used to create a film of desired thickness. The film was left to dry.

EXAMPLE 7

**Mechanical properties**

Compression Module

Compression module of an elastomer (or any other specimen) is the slope of the elastic portion of a stress-strain graph measured when the elastomer is compressed. The table below shows compression module of some elastomeric matrices according to some embodiments of the invention, as function of composition and immersion time in STF (simulated tear fluid). The only difference between the first 6 matrices is the types of PVOH used in the formulations, and their relative amounts. The seventh formulation further differs from the first six in the total amount of PVOH and the various plasticizers.

| Time in STF before the measurement | 100% LCFH | 50% : 50% LCFH : SCPH | 30% : 70% LCFH : SCPH | 70% : 30% LCFH : SCFH | 50% : 50% LCFH : SCFH | 30% : 70% LCFH : SCFH | 50% : 50% LCFH : LCPH |
|---|---|---|---|---|---|---|---|
| 0 min [MPa] | 1.0 | 0.84 | 0.45 | 0.73 | 0.62 | 0.60 | 0.64 |
| 5 min [MPa] | 0.55 | 0.36 | 0.14 | 0.43 | 0.35 | 0.46 | 0.14 |
| 15 min [MPa] | 0.28 | 0.14 | 0.06 | 0.20 | 0.18 | 0.25 | 0.14 |
| 75 min [MPa] | 0.16 | 0.04 | 0.03 | 0.10 | 0.07 | 0.06 | Dissolved |

As can be seen in the table above, in all the matrices, the compression module decreases during immersion in STF. At any given time, when some of the LCFH PVOH is replaced with SCPH PVOH, matrices of different compositions differ from each other in the compression module, with matrices having more SCPH PVOH having lower compression module. When different portions of LCFH PVOH are replaced with SCFH PVOH, there is no clear trend in the differences between the compression modules of the matrices at any given time, and the differences between them are within the estimated error (i.e., the differences are not meaningful).

In some embodiments, the change in compression module is an indication of the degradation of the matrix. Thus, the results, summarized in the above table in regard of matrices in which some of the LCFH PVOH is replaced by SCPH PVOH, demonstrate that the different amounts of the two types of PVOH determine the rate of degradation of these matrices.

The above table also demonstrates that the compression module is not adequate as a measure of the degradation of the other matrices in the table. As will be seen in the next table, for these embodiments, the compression strength is a mechanical property appropriate to demonstrate the matrix degradation.

Compression Strength

Compression strength is the pressure at which a specimen breaks. In a stress-strain graph, it appears as a sharp drop in the graph. The following table shows compression strength results extracted from the same graphs, from which the compression module values presented in the above table have been extracted. The measurements were done using a 10N probe. When the graph showed no sharp drop, the table says "Max Load", and this implies that a 10N load is not sufficient to break the matrix by compression.

| Time in STF before the measurement | 100% LCFH | 50% : 50% LCFH : SCPH | 30% : 70% LCFH : SCPH | 70% : 30% LCFH : SCFH | 50% : 50% LCFH : SCFH | 30% : 70% LCFH : SCFH | 50% : 50% LCFH : LCPH |
|---|---|---|---|---|---|---|---|
| 0 min | Max Load | Max Load | Max Load | Max Load | Max Load | Max Load | Max Load |
| 5 min | | | | | | | |
| 15 min | | | | | | 0.22 | |
| 20 min | | | | | | 0.20 | |
| 30 min | | | | | | 0.12 | |
| 40 min | | | | | 0.22 | 0.09 | |
| 75 min | | | | 0.08 | 0.11 | 0.08 | |

As can be seen in the table, from all tested compositions, only those in which some of the LCFH PVOH is replaced by SCFH PVOH has broken under the 10N load. Among the matrices that broke, the more LCFH PVOH, the longer should the matrix stay in STF in order to break.

In some embodiments, the change in compression strength is an indication of the degradation of the matrix. Thus, the results, summarized in the above table in regard of matrices in which some of the LCFH PVOH is replaced by SCFH PVOH, demonstrate that the different amounts of the two types of PVOH determine the rate of degradation of these matrices.

The above table also demonstrates that the change in the compression strength (at least when measured with a 10N load) is not adequate as a measure of the degradation of the other matrices in the table. As was shown in the preceding table, for those embodiments, the compression module is a mechanical property appropriate to demonstrate the matrix degradation.

Swelling

The swelling of four matrices according to embodiments of the present invention, having formulations like the first four shown in the preceding tables were measured by measuring thickness (i.e., height) and diameter of disk-like matrices before immersion in STF and after different periods of immersion. The matrices were imaged, and the images processed to estimate the thickness and the diameter. The results detailed in the following table show that after 15 minutes (or less) of immersion in STF, all the matrices swelled by no more than 50% $\pm$ 5%.

| Formulation | time [Min] | Thickness % Swelling | Diameter % Swelling | Volume % Swelling |
|---|---|---|---|---|
| LCFH 100% | 2 | 3 | 8 | 19 |
| | 5 | 4 | 11 | 29 |
| | 10 | 9 | 10 | 31 |
| | 15 | 13 | 14 | 47 |
| LCFH 50% SCPH 50% | 2 | 0 | 11 | 20 |
| | 5 | 17 | 9 | 39 |
| | 10 | 24 | 11 | 52 |
| | 15 | 26 | 11 | 55 |
| LCFH 30% SCPH 70% | 2 | 0 | 11 | 20 |
| | 5 | 17 | 9 | 39 |
| | 10 | 8 | 8 | 26 |
| | 15 | 10 | 12 | 38 |
| LCFH 50% | 2 | 0 | 9 | 13 |

(continued)

| Formulation | time [Min] | Thickness | Diameter | Volume |
|---|---|---|---|---|
| | | % Swelling | % Swelling | % Swelling |
| SCFH 50% | 5 | 4 | 8 | 22 |
| | 10 | 13 | 12 | 40 |
| | 15 | 21 | 13 | 54 |

In some embodiments, the maximal swelling observed in the matrices is: At 2 minutes: 21%; At 5 minutes: 40%; At 10 minutes: 52%; At 15 minutes: 55%; At 30 minutes: 75%; All numbers are $\pm 5\%$

Simulated Tear Fluid (STF)

The simulated tear fluid (STF) of the following composition: 6.76 gr NaCl; 2.05 gr sodium bicarbonate; 0.08 gr calcium chloride dehydrate; 1.7 mL acetic acid 10%; in 1 Liter of purified water.

EXAMPLE 8

*Clinical results*

An ophthalmic device comprising equal amounts of long chain fully hydrolyzed PVOH and short chain partially hydrolyzed PVOH with no active pharmaceutical ingredients (API) other than the ophthalmic demulcents (PVOH, glycerol, propylene glycol, and PEG 400) was administered for 3 to 6 hours to each of the participants. The participants were 29 healthy people, (ages 18 to 60). The participants reported the device was almost imperceptible, probably thanks to the degradation properties of the matrix, which allowed the device to fit itself to the individual eye anatomy of the wearer. The devices retained in the eyes, distance visual acuity has not declined, and slip lamp bio-microscopy including fluorescein corneal staining conducted after removing the device from the eye, showed no significant difference from the same tests performed before the administration.

When taken out of the eye, the devices have been softer than before insertion to the eye. Some of the devices were taken out of the eye as crumbs. Some of the devices were dried and weighed after being taken out of the eye. Their weight was found to be about 70% lower than the weight of the device before it was inserted to the eye.

Dry Eye disease

First in human

Nearly 40 healthy subjects experienced using ophthalmic devices according to the invention for 6 hours. All reported that the device was easy to use and almost imperceptible, probably thanks to the degradation properties of the matrix, which allowed the device to fit itself to the individual eye anatomy of the wearer. No serious adverse events were observed during the study. Few participants experienced mild discomfort or tearing in the initial few minutes after the application of the device, which disappeared spontaneously later on (within 15 minutes).

According to Visual Analogue Scale (VAS) questionnaires, wear of the device over time was mostly imperceptible and no discomfort was reported at the end of the treatment before removal of the device. The device was found safe for use.

During the treatment, there were reports of enhanced moisture in the treated eye, improved feeling (eyelid easily sliding), relaxation of the eye, and improved or clear vision, probably related to tear film stabilization. In some subjects, who reported some level of dryness in their eyes at baseline, there was an improvement in their dryness symptoms. Some subjects applied the device independently, and most subjects easily removed the device by themselves.

When taken out of the eye, the devices have been softer than before insertion to the eye. Some of the devices were taken out of the eye as crumbs. Some of the devices were dried and weighed after being taken out of the eye. Their weight was found to be about 70% lower than the weight of the device before it was inserted to the eye.

5 days trial

10 patients suffering from mild to moderate dry eye disease took part in a clinical trial, in which each patient inserted under the lower eyelids of both eyes an ophthalmic device according to an embodiment of the present invention; consisting essentially of water, PVOH, and plasticizers Once daily for 5 consecutive days.

All the participants were trained to apply the device by themselves. All participants reported that the device was easy to use and almost imperceptible, probably thanks to the degradation properties of the matrix, which allowed the device to fit itself to the individual eye anatomy of the wearer. No serious adverse events were observed during the study. Few participants experienced mild discomfort or tearing in the initial few minutes after the application of the device, which disappeared spontaneously later on, within 15 minutes from application. No serious adverse events were observed throughout the 5-day study. According to Visual Analogue Scale (VAS) questionnaires, wear of the device over time was mostly imperceptible, at least 15 minutes from the beginning of the daily treatment until removal of the device. The device was found safe for use.

When taken out of the eye, the devices have been softer than before insertion to the eye. Some of the devices were taken out of the eye as crumbs. Some of the devices were dried and weighed after being taken out of the eye. Their weight was found to be about 70% lower than the weight of the device before it was inserted to the eye.

Presbyopia

A total of 18 subjects diagnosed with presbyopia, aged 45-61 were enrolled in the study. All subjects completed the study as planned. Following insertion of a device according to an embodiment of the invention, comprising 200mcg of pilocarpine, under an eyelid of one of the eyes, mean pupil diameter decreased compared to the untreated eye, the mean minimum pupil diameter in the treated eye observed 1 hour after insertion. The mean pupil diameter in the treated eye increased over time up to 8 hours post-insertion. Two hours after insertion, monocular near visual acuity measured using the Jaeger chart decreased to J1 in all subjects but one, who had J1+. BCDVA remained at 6/6 during the study.

All participants reported that the device was easy to use and almost imperceptible, probably thanks to the degradation properties of the matrix, which allowed the device to fit itself to the individual eye anatomy of the wearer. No serious adverse events were observed during the study. Some subjects reported minor discomfort for up to 5 minutes after insertion. All subjects reported that the device was easy to use and almost imperceptible. IOP remained within normal range during the study. No clinically significant changes in slit lamp bio-microscopy were observed between baseline and endpoint.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

It is the intent of the applicant(s) that all publications, patents and patent applications referred to in this specification are to be incorporated in their entirety by reference into the specification, as if each individual publication, patent or patent application was specifically and individually noted when referenced that it is to be incorporated herein by reference. In addition, citation or identification of any reference in this application shall not be construed as an admission that such reference is available as prior art to the present invention. To the extent that section headings are used, they should not be construed as necessarily limiting. In addition, any priority document(s) of this application is/are hereby incorporated herein by reference in its/their entirety.

The disclosure of this application also includes the following numbered clauses:

Clause 1. An elastomeric matrix, comprising:

    a. poly(vinyl alcohol) (PVOH);
    b. one or more organic plasticizers; where a combined mass ratio of said one or more organic plasticizers to said PVOH is at least 2:1; and
    c. water;

wherein said PVOH comprises PVOH of two or more types that differ from one another in one or both of hydrolysis degree (HD) and chain length.

Clause 2. The elastomeric matrix according to clause 1, wherein said PVOH comprises PVOH of two or more types that differ from one another in hydrolysis degree (HD).

Clause 3. The elastomeric matrix according to clause 1 or clause 2, wherein two types of the PVOH differ in chain length by at least 1000, and have similar degrees of hydrolysis, both between 97% and 100%.

Clause 4. The elastomeric matrix according to any one of clauses 1-3, wherein a first type of said two or more types has a hydrolysis degree from 97 % to 100 %.

Clause 5. The elastomeric matrix according to any one of clauses 1-4, wherein a second type of said two or more types has a hydrolysis degree is lower than 93%.

Clause 6. The elastomeric matrix according to any one of clauses 1-5, wherein a second type of said two or more types has a hydrolysis degree from 80 % to 93%.

Clause 7. The elastomeric matrix according to any one of clauses 1-6, wherein a first type of said two or more types has chains with more than 2500 units.

Clause 8. The elastomeric matrix according to any one of clauses 1-7, wherein a second type of said two or more types has chains with less than 1500 units.

Clause 9. The elastomeric matrix according to any one of clauses 1-8, wherein a relation between a first type of PVOH and a second type of PVOH of said two or more types is from about 3:1 to about 1:3.

Clause 10. The elastomeric matrix according to any one of clauses 1-9, wherein a first type of said two or more types has chains with more than 2500 units and hydrolysis degree of 97% to 100%, and a second type of said two or more types has chains with less than 1000 units and hydrolysis degree of 80% to 93%.

Clause 11. The elastomeric matrix according to clause 3, wherein said second type of PVOH makes more than 50% of said PVOH.

Clause 12. The elastomeric matrix according to any one of clauses 1-11, wherein said at least two types of PVOH determine a time of degradation of said elastomeric matrix.

Clause 13. The elastomeric matrix according to clause 12, wherein said degradation comprises change in mechanical properties under wet conditions.

Clause 14. The elastomeric matrix according to clause 12, wherein said degradation comprises change in shape under wet conditions.

Clause 15. The elastomeric matrix according to any one of clauses 1-14, wherein said at least two types of PVOH determine a time development of mechanical properties of said elastomeric matrix under wet conditions.

Clause 16. The elastomeric matrix according to any one of clauses 1-15, wherein a combined mass content of said PVOH and said one or more organic plasticizers is at least 70 %wt of the total weight of the matrix excluding said water.

Clause 17. The elastomeric matrix according to any one of clauses 1-16, wherein said elastomeric matrix is used as an ophthalmic device.

Clause 18. The elastomeric matrix according to any one of clauses 1-17, wherein said combined mass ratio of said one or more organic plasticizers to said PVOH is less than 20:1.

Clause 19. The elastomeric matrix according to any one of clauses 1-18, characterized by substantially isotropic swelling and shrinking.

Clause 20. The elastomeric matrix according to any one of clauses 1-19, wherein said elastomeric matrix swells by less than 50 % by volume under wet conditions.

Clause 21. The elastomeric matrix according to any one of clauses 1-20, wherein said one or more organic plasticizers is independently selected from the group consisting of a polyol, a polyprotic organic acid, a polyamine, an alkyl gluceth, an aliphatic polyalkylene glycol, an ethanolamine, a saccharide, an oligosaccharide, an amino acid, a polyphenol, tromethamine, urea, tannic acid, and any salt thereof and/or combinations thereof.

Clause 22. The elastomeric matrix according to clause 21, wherein said polyol is selected from the group consisting of ethylene glycol, diethylene glycol, triethylene glycol, tetraethylene glycol, propylene glycol, triacetin erythritol, a poly-glycol, a poloxamer, and a co-polymer thereof, and glycerol and esters thereof.

Clause 23. The elastomeric matrix according to clause 21, wherein said polyprotic organic acid is selected from the group consisting of oxalic acid, maleic acid, citric acid, and any salt thereof.

Clause 24. The elastomeric matrix according to clause 21, wherein said polyamine is selected from the group consisting of spermine, spermidine, diethylenetriamine, triethylenetetramine, tris(2-aminoethyl)amine, a polyethylenimine, and any salt thereof.

Clause 25. The elastomeric matrix according to clause 21, wherein said aliphatic polyalkylene glycol is selected from the group consisting of a polyethylene glycol, a polypropylene glycol, a poly-glycol, a poloxamer, and a polysorbate.

Clause 26. The elastomeric matrix according to clause 21, wherein said aliphatic polyalkylene glycol is polyethylene glycol.

Clause 27. The elastomeric matrix according to clauses 24-25, wherein a mass content of said PVOH is substantially equal to a mass content of said aliphatic polyalkylene glycol.

Clause 28. The elastomeric matrix according to clause 27, wherein said aliphatic polyalkylene glycol is selected from the group consisting of polyethylene glycol, polypropylene glycol, and any mixture thereof.

Clause 29. The elastomeric matrix according to any one of clauses 1-28, wherein said water constitute less than 50 % wt of the total mass content of the matrix.

Clause 30. The elastomeric matrix according to any one of clauses 1-29, wherein said mass content of said PVOH is less than 25 %wt of the total weight of non-water ingredients of the matrix.

Clause 31. The elastomeric matrix according to any one of clauses 1-30, wherein said mass content of said PVOH is less than a third of the total weight of said one or more organic plasticizers.

Clause 32. The elastomeric matrix according to any one of clauses 1-31, essentially devoid of covalent crosslinking.

Clause 33. The elastomeric matrix according to any one of clauses 1-32, further comprising a pharmaceutically active agent.

Clause 34. An ophthalmic device comprising the elastomeric matrix according to clause 1.

Clause 35. The ophthalmic device according to clause 34, wherein said elastomeric matrix consists of ophthalmically acceptable ingredients.

Clause 36. The ophthalmic device according to clause 34 or clause 35, wherein said device weights between 1 mg and 50 mg or between 1mg and 30 mg.

Clause 37. The ophthalmic device according to any one of clauses 34-36, wherein said elastomeric matrix comprises a pharmaceutically active agent.

Clause 38. The ophthalmic device according to any one of clauses 34-37, wherein said ophthalmic device is configured to be positioned on a surface of an eye.

Clause 39. The ophthalmic device according to clause 38, wherein said surface is at least partially underneath at least one of the upper eyelid, the lower eyelid and outside a cornea of the eye.

Clause 40. The ophthalmic device according to any one of clauses 34-39, wherein said ophthalmic device is configured for delivering at least one pharmaceutically active agent to an eye for an extended period of time of between 5 minutes and 24 hours.

Clause 41. A medical device comprising two elastomeric matrices, each according to clause 1, wherein the two matrices differ from one another in one or more of:

a. ratio between the mass content of PVOH included in the matrix and the mass content of the matrix;

34

b. ratio between the mass content of PVOH included in the matrix and the combined mass contents of the one or more plasticizers;
c. the types of PVOH; and
d. mass ratios between the types of PVOH.

Clause 42. The medical device according to clause 41, wherein the two matrices touch across a touching surface.

Clause 43. The medical device according to clause 42, wherein the touching surface is a closed surface.

Clause 44. The medical device according to any one of clauses 41-43, wherein the two matrices degrade under wet conditions at different rates.

Clause 45. The medical device according to any one of clauses 41-44, wherein the two matrices show distinct mechanical properties under dry conditions.

Clause 46. An elastomeric matrix, comprising:

a. poly(vinyl alcohol) (PVOH);
b. one or more organic plasticizers; where a combined mass ratio of said one or more organic plasticizers to said PVOH is at least 2:1; and
c. water;

wherein said PVOH comprises only PVOH of chains shorter than 2000 units or shorter than 1000 units and degree of hydrolysis smaller than 90%, and
said water makes less than 10% or less than 5% of the matrix.

**Claims**

1. An elastomeric matrix for use in treating an ophthalmic symptom, disease or disorder, **characterized in that** the elastomeric matrix comprising:

   a. poly(vinyl alcohol) (PVOH);
   b. one or more plasticizers, none of which is water, each **characterized by** exhibiting at least two hydrogen bond forming functional groups; where a combined mass ratio of said one or more plasticizers to said PVOH is at least 2:1 and less than 30: 1;
   c. one or more active pharmaceutical ingredients (APIs); and
   d. water.

2. The elastomeric matrix for use according to claim 1, wherein said PVOH comprises PVOH of two or more types that differ from one another in one or both of hydrolysis degree (HD) and chain length.

3. The elastomeric matrix for use according to claim 1 or claim 2, wherein said water constitutes less than 50 %wt of the total mass content of the matrix.

4. The elastomeric matrix for use according to any one of claims 1-3, wherein one of the APIs is pilocarpine.

5. The elastomeric matrix for use according to any one of claims 1-4, wherein one of the APIs is for lowering the intraocular pressure of the eye.

6. The elastomeric matrix for use according to any one of claims 1-5, wherein the ophthalmic symptom, disease, or disorder is presbyopia.

7. The elastomeric matrix for use according to any one of claims 1-6, wherein the ophthalmic symptom, disease, or disorder is dry eye.

8. The elastomeric matrix for use according to any one of claims 1-7, wherein one of the APIs is for treating dry eye.

9. An elastomeric matrix for use in treating dry eye, **characterized in that** the elastomeric matrix comprising

    a. poly(vinyl alcohol) (PVOH);
    b. one or more plasticizers, none of which is water, and each **characterized by** exhibiting at least two hydrogen bond forming functional groups; where a combined mass ratio of said one or more plasticizers to said PVOH is at least 2:1 and less than 30:1; and
    c. water.

10. The elastomeric matrix for use in treating dry eye according to claim 9, wherein said PVOH comprises PVOH of two or more types that differ from one another in one or both of hydrolysis degree (HD) and chain length.

11. The elastomeric matrix for use in treating dry eye according to claim 9 or claim 10, wherein said water constitutes less than 50 %wt of the total mass content of the matrix.

12. The elastomeric matrix for use according to any one of claims 9-11, consisting essentially of the PVOH, water, and one or more plasticizers.

13. An elastomeric matrix comprising:

    a. poly(vinyl alcohol) (PVOH);
    b. one or more plasticizers, none of which is water, and each **characterized by** exhibiting at least two hydrogen bond forming functional groups; where a combined mass ratio of said one or more plasticizers to said PVOH is at least 2:1 and less than 30:1;
    c. an active pharmaceutical ingredient which is one or more of an anti-inflammatory agent, a corticosteroid, a non-steroidal anti-inflammatory agent, an antibiotic agent, an antihistamine agent, a carbonic anhydrase inhibitor, a beta-adrenergic receptor antagonist, a prostaglandin, a prostaglandin analogue, a prostamide, a lubricant, a fatty acid, a lipid, an anticholinergic agent, or a mydriatic agent;
    and
    d. water;
    wherein said PVOH comprises PVOH of two or more types that differ from one another in one or both of hydrolysis degree (HD) and chain length, and
    wherein said water constitutes less than 50 %wt of the total mass content of the matrix.

14. The elastomeric matrix according to claim 13, wherein the one or more active pharmaceutical ingredient is Azithromycin, Bimatoprost, Cetyl alcohol, Cyclosporine, Dorzolamide, Doxycycline, Fluoromethalone, Ketorolac, Latanoprost, Lifitegrast, Loteprednol, Olopatadine, Pilocarpine, Prostamide, Silicone oil, Stearyl alcohol, Tafluprost, Tetracycline, or Travoprost.

15. The elastomeric matrix according to any preceding claim, wherein each of the one or more plasticizers is organic.

Figure 1

Figure 2

Figure 3

Figure 4a

Figure 4b

Figure 5

Figure 6

Figure 7

Figure 8

Figure 9

Figure 10

Figure 11a

Figure 11b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63462269
- US 63463034 B
- US 4874562 A
- US 4663358 A

- US 10513588 B
- WO 2022016268 A
- US 20220168142 A1
- US 20170226298 A1